# EUROPEAN PATENT APPLICATION

(11) **EP 2 704 084 A1**
(43) Date of publication of application: **05.03.2014**
(21) Application number: 12773747.6
(22) Date of filing: 21.04.2012
(51) Int. Cl.: G06Q 30/02

(54) **SYSTEM FOR EXAMINING AND PROVIDING CONTACT LENSES**

(30) Priority: 22.04.2011 JP 2011095979
(71) Applicant: Matsuba, Hiroo, Kobe-shi, Hyogo 650-0021 (JP)
(72) Inventor: Matsuba, Hiroo, Kobe-shi, Hyogo 650-0021 (JP)
(74) Representative: Horn Kleimann Waitzhofer
(86) International application number: PCT/JP2012/060799
(87) International publication number: WO 2012/144642

(57) **Abstract**

The present invention is a system for determining the permission of the home delivery service when a user demands the home delivery contact lens renewal. The system comprises a server terminal 10 utilized by the system control administrator, a vendor client terminal 20, a distributor client terminal 30 and a user client terminal 40. The server terminal 10 comprises a membership fee payment status managing part 12 for managing the membership fee payment of the user; a user information managing part 13 for managing the diagnosis information and the contact lens information; a renewal period managing part 14 for informing the forthcoming contact renewal due date based on the membership fee payment information and the diagnosis information and the contact lens information; and a renewal guiding part 15 for displaying the selectable contact lens information and the contact lens distributor based on the user demand information and the diagnosis information. If the user demands the home delivery service, a home delivery renewal permission determination part 151 determines the permission of home delivery contact lens renewal, and if it is permitted, guide information is displayed via the renewal guiding part 15. If it is not permitted, guide information for visiting the contact lens distributor store and taking eye examination is displayed.

## Description

### Technical Field

This invention relates to a contact lens delivery system and an eye examination guiding system and a method of using the same, which can coordinate the appropriate renewal period for the current disposable contact lens to an appropriate disposable contact lens based on the appropriate eye examination and prescription by the oculist for the people using the disposable contact lens having a limited usable period such as "one-day", "two-week" and so on.

### Background Art

A contact lens is categorized as the medical device for correction of myopia, hyperopia and astigmatism.

The contact lens was classified into the "medical implement" before the enforcement of the "Revised Japanese Pharmaceutical Law" since April 2005. However it has classified into the "medical device" by the enforcement of the "Revised Japanese Pharmaceutical Law" on April 2005. Especially, the contact lens is classified into class III "Highly controlled medical devices" requiring the especially highly controlled management. This classification is based on the contact lens characteristics of being used for a long term while attached to the delicate membrane of a cornea which should be kept transparent. So the contact lens should be highly controlled. If not, it can cause severe detriment to the user eye health. Therefore, the user should take an appropriate eye examination by the oculist before wearing the contact lens and the contact lens should be provided by the appropriate prescription by the oculist. The retailers of the contact lens have a legal obligation to sell according to the legal prescription and to post the sales management staff at the counter.

There are various types of the contact lens. For example, the contact lens is classified into the myopia-use type, the hyperopia-use type, astigmatism-use type and the presbyopia-use type according to its purpose, and the contact lens uses various types of structure and material. In addition, there are many contact lens suppliers and, various types of the contact lens products are developed by the vendors day by day.

As a hard contact lens material, an oxygen non-permeable contact lens (PMMA resin: poly-methyl methacrylate resin) and an oxygen permeable contact lens (02 lens) are known.

As a soft contact lens material, hydrous material employing a gelatinous, synthetic polymeric compound such as Poly-HEMA (Poly-hydroxyl-ethyl methacrylate) or poly-vinyl-pyrrolidone is mainly used. For example, there are two types in soft contact lens. One is "the disposable replacement type". A user can use this type during a prescribed period such as one-day and two-week without any maintenance. The other is "the maintenance and renewal type". A user can use this type during a prescribed period such as two-week, one-month and three-month with lens maintenance treatment and the user will renew from the current contact lens to a new soft contact lens when the renewal period comes.

As described above, the contact lens is classified into the "Highly controlled medical devices". It is important for the user to judge whether the contact lens is the suitable type for the user or not, and to select the contact lens having the appropriate curve and optical property when the user purchases the contact lens.

This judgment and selection required in the contact lens purchasing should be determined by the oculist who has high professional skill and experience in the contact lens field. The information such as the diagnosis examined by the oculist in the past and the user's experience with the contact lens are very useful information for judging the user aptitude for wearing the contact lens, and for selecting the appropriate contact lens for the user and for judging the renewal period of the contact lens. Therefore, it is preferable that such user information regarding the contact lens is collected and managed on one site.

Recently, the renewal type contact lens has become popular and widely spread. However, the eyesight problem such as the dry-eye occurs depending on user physical constitution and user condition. Therefore, it is not sure that the contact lens condition is maintained in good condition even in the usable term before the due date of the periodical renewal of the contact lens.

The renewal on demand system is known in the prior art. This system can achieve the renewal from current contact lens to a high oxygen permeable contact lens or a new contact lens on demand freely even in the usable term before the due date of the periodical renewal of the current contact lens. However, this system is based on the membership fee and the distributable contact lens is limited to a particular contact lens vendor (JP No.3731003).

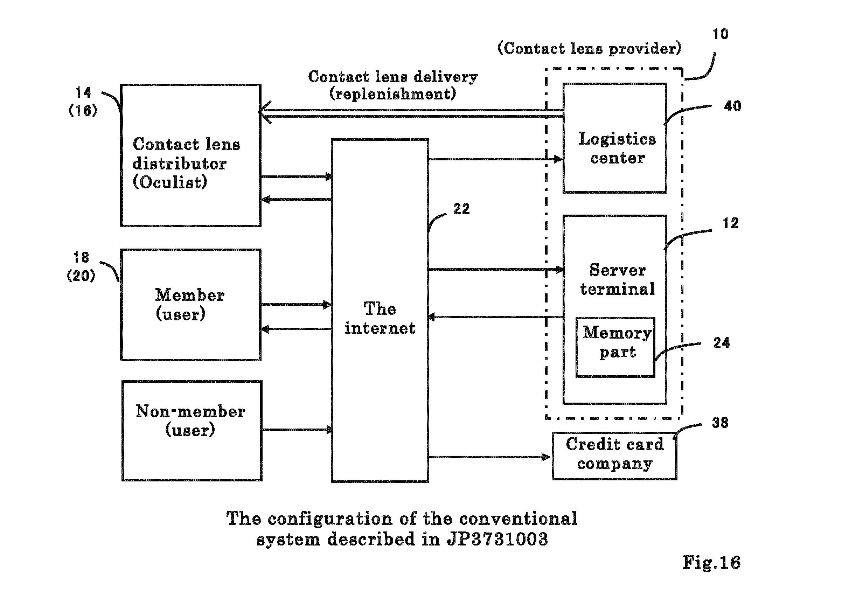

As shown in Fig.16, the system described in JP No.3731003 provides the contact lens having predetermined periodical renewal time to the user, and assists the user to take the eye examination. The system comprises a server terminal utilized by the contact lens provider, a memory portion used by the server terminal, a client terminal utilized by the affiliated store which can provide the eye examination, a membership fee payment status management portion and a determination means for permitting the contact lens delivery. The client terminal sends the user personal data including the information of the user eye diagnosis and information of the prescribed contact lens and the information of the user identification via the computer network to the server terminal, and the collected information is stored in the memory. The membership fee payment status management portion inputs the information of the periodical membership fee payment to the contact lens provider from each user to the server terminal, and each collected information is stored with user identification information in the memory.

The determination means for permitting the contact lens providing sends the permission signal for contact lens delivery from the server terminal to the client terminal installed in the affiliated store via the computer network. The permission signal is generated on the condition that the user takes the periodical eye examination by the oculist assured by the user eye diagnosis information stored in the memory in addition to the user status of the membership fee payment.

According to the conventional system described in JP No.3731003, the user who has been paying the periodical membership fee can renew the current use contact lens to the new contact lens by visiting the affiliated store if the current use contact lens condition is deteriorated. If the renewal due date comes, the user will visit the affiliate store by himself and renew the current contact lens to the new contact lens. If he does not renew to the new contact lens, he has to keep on using the current contact lens until he visits the affiliate store by himself to renew to the new contact lens even if he keeps on paying the membership fee. When the user visits the affiliate store for renewal, the user should take an eye examination, so the periodic eye examination is secured. By this conventional system, there is a merit for user to use the contact lens in good condition; there is a merit for contact lens vendor to promote the contact lens periodical renewal; and there is a merit for the affiliate store to assure the user's periodic eye examination.

Patent prior art 1: JP 3731003

### Disclosure of the invention

### The problems to be solved

The conventional system described in the prior art JP 3731003 has the following problem. According to the conventional system described in the prior art JP 3731003, the replaceable contact lens products are limited to the particular contact lens vendor, so it cannot apply to the multivendor system and multi-supplier system cooperating with various contact lens affiliate stores in various areas.

Recently, the contact lens products and the distribution channels of the contact lens are varied corresponding to the expansion of the contact lens market. The life styles of the users are also varied, and many users demand the newly developed various contact lens of various vendors without being bound to a particular vendor and a particular contact lens product. However, the contact lens providers in the conventional system utilizing the membership fee payment system try to keep on limiting the users. Therefore, there is no choice for the user but to keep on using the same contact lens product supplied by the particular contact lens provider. It is quite difficult for the user to change to other contact lens products supplied by other vendors.

Recently each contact lens vendor can provide the good quality contact lens by researching and developing through their own effort, so many users think of the contact lens as mass consumption products. The oculists and the contact lens stores are required for corresponding to such diverse demand of the user. However, the conventional system does not apply to the multi-vendor circumstances for the user needs and the contact lens vendor effort. The conventional system does not satisfy the diverse user needs for wearing the various contact lens by TPO (time, place, opportunity) according to the user life style, without staying with the particular contact lens product supplied by the particular vendor. To solve this problem, the new system which can achieve the cooperation relationship across different plural contact lens vendors and different plural contact lens distributors is required. However, the conventional system disclosed in the JP No.3731003 is limited to the particular contact lens product supplied by the particular vendor, and it is not available to the multivendor and multi-supplier system.

Mr. Hiroo Matsuba who is the inventor of this invention has worked for development and distribution of the contact lens and contributed to the contact lens health services from the early stage of the Japanese contact lens market. Mr. Hiroo Matsuba guides the contact lens distributor to provide the appropriate eye examination to the user. Mr. Hiroo Matsuba reached this invention by considering the necessity of the new system that can apply to the multivendor system across different plural contact lens vendors and different plural contact lens distributors for corresponding to the diverse of the user life style and the newly developed varieties of contact lens.

Therefore, it is an object of the present invention to provide a system that can utilize the merit of the user payment of the membership fee system and can be applied to the multivendor system across different plural contact lens vendors and different plural contact lens distributors, and which can provide the user contact lens always in good condition, especially which can apply to the disposable type or the renewal type contact lens distribution by utilizing the home delivery service while securing the appropriate eye examination.

### Means for solving the problems

In order to achieve the above-mentioned object, the present invention comprises the following configuration.

The following configuration can be applied in various combinations. The examples and the technical features are not limited to the configuration shown in the following description, but are described in the whole of this application and drawings and are recognized by the invention understood from the whole of this application and drawings.

This invention of the contact lens delivery system and an eye examination guiding system can be applied to both the case the case that there is single contact lens vendor, and the case that the case that there are plural contact lens vendors.

First in the case that there is single contact lens vendor, the system configuration is described as follows.

The contact lens delivery system and the eye examination guiding system for managing the delivery of the contact lens which has predetermined validation period for renewal and guiding the user for taking the eye examination, comprises; a server terminal utilized by a system control administrator; a vendor client terminal used by a contact lens vendor; distributor client terminal used by each contact lens distributor; user client terminal used by each user; a computer network providing data connection among those terminals; a user information managing part for processing the user information based on the diagnosis information obtained by the user eye examination and the prescribed contact lens information with referencing to the user personal data, and storing the processed data in the memory part of the server terminal; a renewal period managing part for informing the user client terminal of the forthcoming contact lens renewal due date based on the diagnosis information and the prescribed contact lens information stored in the memory part; a user demand information input part for receiving the user demand regarding the contact lens renewal from the user client terminal; a home delivery renewal permission determination part for judging the permission of the utilization of the home delivery contact lens renewal without diagnosis based on the user eye status indicated in the latest diagnosis information and the interval from the previous renewal date; a renewal guiding part for displaying the information of the replaceable contact lens products by the home delivery replacement on the user client terminal on the condition that the home delivery renewal permission determination part judges the permission of the home delivery contact lens renewal when the user demand for the home delivery is included in the user demand information.

According to the above configuration, the system can judge and guide the home delivery contact lens renewal without the oculist eye examination is available or the home delivery contact lens renewal is available on the condition that user takes the oculist eye examination at the contact lens distributor depending on the user's eye condition. For this operation, the system can enhance the convenience for user. Appropriate judgment process for permission of the home delivery contact lens renewal without oculist eye examination is conducted by the home delivery renewal permission determination part based on the user eye condition and the interval term from the previous renewal date. Therefore, the system can guide an appropriate operation such as the guiding of the refusal for the home delivery contact lens renewal based on the user eye condition or guiding of shortening the term up to the next contact lens renewal due date if the home delivery contact lens renewal is permitted this time.

Another system configuration, in which plural contact lens vendors join this system and the user can exchange the different contact lens provided by the different vendor corresponding to the user demand, is described as follows.

The contact lens delivery system and the eye examination guiding system for managing the delivery of the contact lens which has predetermined validation period for renewal and guiding the user for taking the eye examination, comprises; a server terminal utilized by a system control administrator; a vendor client terminal used by each contact lens vendor; a distributor client terminal used by each contact lens distributor; a user client terminal used by each user; a computer network providing data connection among those terminals; a user information managing part for processing the user information based on the diagnosis information obtained by the user eye examination and the prescribed contact lens information with referencing to the user personal data, and storing the processed data in the memory part of the server terminal; a renewal period managing part for informing the user client terminal of the forthcoming contact lens renewal due date based on the diagnosis information and the prescribed contact lens information stored in the memory part; a user demand information input part for receiving the user demand regarding the contact lens renewal including the replacement information for desired vendor and the desired product from the user client terminal; a home delivery renewal permission determination part for judging the permission of the utilization of the home delivery contact lens renewal without diagnosis based on the user eye status indicated in the latest diagnosis information and the interval from the previous renewal date; a renewal guiding part for displaying the information of the replaceable contact lens product of each vendor based on the latest diagnosis and the user demand information regarding the contact lens renewal on the user client terminal on the condition that the home delivery renewal permission determination part judges the permission of the home delivery contact lens renewal when the user demand for the home delivery contact lens renewal in the user demand information.

According to the above configuration, the system can connect the various terminals such as plural contact lens vendors, plural contact lens distributors (in which the eye examination is available) and users who pay the membership fee periodically to the system control administrator. In addition, according to the above configuration, when the system receives the input of the user demand information regarding the contact lens renewal including the replacement information for the desired contact lens vendor and desired contact lens product, the system can display and guide the information regarding the contact lens distributor location, the selectable contact lens vendor and the selectable contact lens product.

The first judgment rule managed by the home delivery renewal permission determination part is the judgment based on the interval term up to the recommended renewal due date expected from the previous renewal date. For example, the home delivery renewal permission determination part displays the inquiry how the user use the current contact lens on the user client terminal when the length of the interval term from the previous renewal date is longer or shorter than the expecting interval term, and receives the answer from the user to judge the permission of the home delivery contact lens renewal considering the answer for information of how the user uses the current contact lens.

The second judgment rule managed by the home delivery renewal permission determination part is the judgment based on the user's use experience of the desired contact lens product based on the prescription. For example, the home delivery renewal permission determination part accesses and references the prescribed contact lens information managed by the user information managing part and judges the permission of the home delivery renewal on the condition that the user's desired product is the contact lens product that was prescribed to the user before. It is preferable that the renewal term of the contact lens (one-day disposal, one-week disposal, two-week disposal or one-month disposal) is added to the judgment rule.

The third judgment rule managed by the home delivery renewal permission determination part is the judgment based on whether desired contact lens product is from the vendor who the user has used for other products before. For example, the home delivery renewal permission determination part accesses and references the prescribed contact lens information managed by the user information managing part and judges the permission of the home delivery renewal on the condition that user take the oculist eye examination at the contact lens distributor store if the user desired product is not the contact lens product whose vendor the user has used for other products before.

The fourth judgment rule managed by the home delivery renewal permission determination part is the judgment from the validity of the prescription issued by the oculist. For example, the home delivery renewal permission determination part manages the validity of the prescription by the oculist and judges the permission of the home delivery renewal on the condition that the user takes a new eye examination at the contact lens distributor store, if there is the demand for the home delivery contact lens renewal included in the user demand information and if the validity period is over from the latest eye examination by the oculist.

The fifth judgment rule managed by the home delivery renewal permission determination part is the judgment from the user eye condition, especially the density of the corneal endothelium cell count of the user's eye.

For example, the home delivery renewal permission determination part manages the threshold value of the density of the corneal endothelium cell count of user eye for permitting the home delivery contact lens renewal and judges the permission of the home delivery renewal on the condition that the density of the corneal endothelium cell count of user eye equals to or is larger than the threshold, if there is the demand for the home delivery contact lens renewal included in the user demand information.

Next, this system can utilize the inventory information of the contact lens distributor. The contact lens delivery system and the eye examination guiding system can include an inventory managing part for managing the inventory information of the each contact lens product of each contact lens vendor. The renewal guiding part can utilize the inventory information for determining the selection of the contact lens distributor. In the actual distribution market, many contact lens distributors are in the affiliate chain of the particular contact lens vendor. Some of the distributors might provide products beyond the affiliate chain, but in most cases, the contact lens distributors do not keep enough stock for other contact lens vendors' products in inventory. In addition, contact lens distributors may run out of stock for the well-selling product even it is provided by the contact lens vendor in the affiliate chain. If the user visits the contact lens distributor store, and finds that the desired contact lens product runs out of stock, the user satisfaction decreases. However, this system can display and guide the selectable contact lens vendor and the selectable contact lens product in the contact lens distributor store by cooperating with the inventory managing part. Therefore, such problem does not occur.

Next, as a merit of the membership fee payment system, the user can require the contact lens renewal freely regardless of the normal periodical renewal due date comes or not. The contact lens delivery system and the eye examination guiding system can include a renewal requirement input part for inputting the renewal requirement information to require the contact lens renewal regardless of whether the normal periodical renewal due date comes or not, if the renewal guiding part receives the renewal requirement information, the renewal guiding part displays and guides the information of the contact lens distributors and contact lens products corresponding to the user is voluntarily renewal requirement.

This system can utilize the area information that indicates the demand of the user of the contact lens distributor location area. The contact lens delivery system and the eye examination guiding system can accept the demand for the area of the contact lens distributor location as one of the user demand information. The user demand information input part utilizes the area information stored in the memory part of the server terminal for listing up the selectable contact lens distributors in the desired area.

From the view point of the user, the convenience area may change. If the desired visiting day to the contact lens distribution store is a week day, the area near the office will be fine. If it is a week-end day, the area near the home will be fine. If it is a day during the business trip schedule, the area near the business trip location will be fine. The present system can enhance the convenience of the area selection by displaying the selectable contact lens distributor with the area information.

From the view point of the contact lens distributor, if an unknown user comes to the store unexpectedly, the contact lens distributor does not know the user historical circumstances. The present system can provide various user information to the contact lens distributor in advance. For example, the present system can provide the latest user diagnosis information and the user demand information from the server terminal to the contact lens distributor who is selected as the contact lens distributor for the forthcoming contact lens renewal guided by the renewal guiding part.

Next, the present system can cooperate with the insuree qualification information of the public health insurance. For example, the contact lens delivery system and the eye examination guiding system further comprises an insuree qualification information managing part for storing the insuree qualification information of the national health insurance to the memory part with referencing to the user's other information. The renewal period managing part informs the forthcoming contact lens renewal due date to the user client terminal on condition that verifying the no suspension of the insuree qualification. According this present invention, if the user selects a purchase plan utilizing the national health insurance, the system can confirm his status and guide that this contact lens renewal plan can be utilized on the condition that there is no suspension of the insuree qualification. Therefore, the problem that the user cannot use the national health insurance does not occur with the present system.

As the further development, if the insuree qualification information managing part cannot verify the no suspension of the insuree qualification of the national health insurance, the renewal period managing part can display the information for guiding the payment of the national health insurance premium instead of displaying the information for guiding the contact lens renewal.

Next, the present system can display the contact lens product information to the user in order to enhance the user convenience to replace the current contact lens product with a new contact lens product among various contact lens vendors and various contact lens distributors, not fixed to a particular contact lens product. For example, the configuration is modified as follows. The vendor client terminal includes a product information providing part for providing the information of the current contact lens products line-up and the newly developed contact lens products line-up to the server terminal. The server terminal includes product information managing part for managing the contact lens product information provided by the vendor client terminal. The renewal period managing part informs the forthcoming contact lens renewal due date and the renewal guiding part displays the product information of the recommended contact lens products based on the diagnosis information.

According to the above configuration, the present system can guide and inform the user of the selectable contact lens product recommended from the view point of the oculist based on the user diagnosis information, not stereotyped advertisement of the newly developed contact lens products

### Effect of the invention

According to the above-mentioned configuration of the invention of a contact lens delivery system and an eye examination guiding system, the multivendor system across different plural contact lens vendors and different plural contact lens distributors is achieved on the basis of the user payment of the membership fee system, the user can use the contact lens always in good condition while securing the appropriate eye examination even if the user utilizes the home delivery service.

### Detailed description of the preferred embodiment

Some embodiments of a contact lens delivery system and an eye examination guiding system according to the present invention are described below with reference to the relevant drawing. Needless to add, the claims of the present invention include but are not limited to the configuration shown in the following embodiments.

### Embodiment 1

The embodiment 1 of the configuration of the contact lens delivery system and the eye examination guiding system 100 is described below.

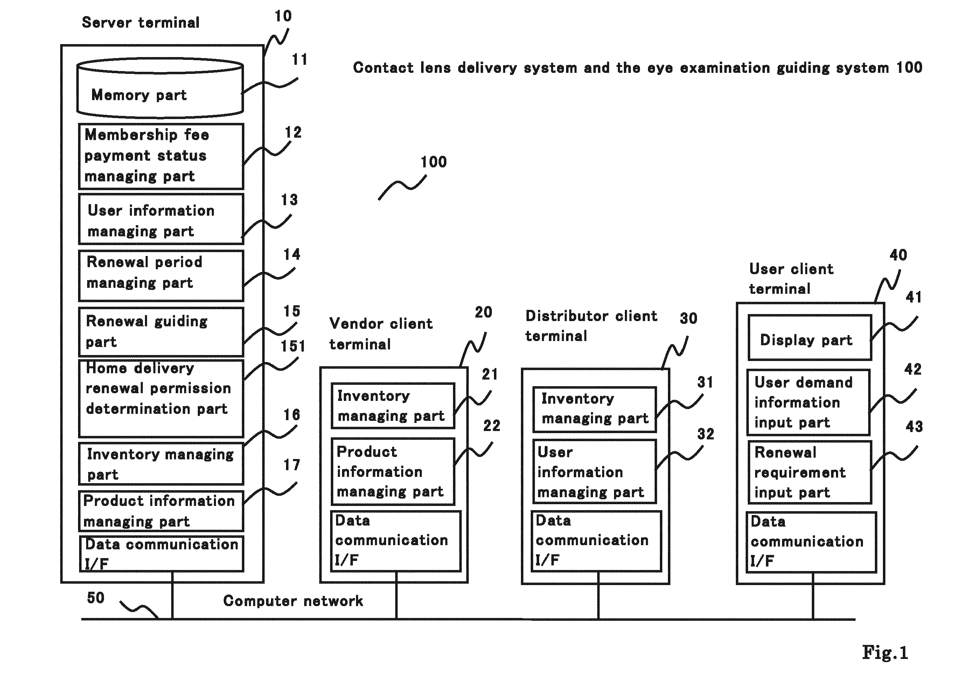

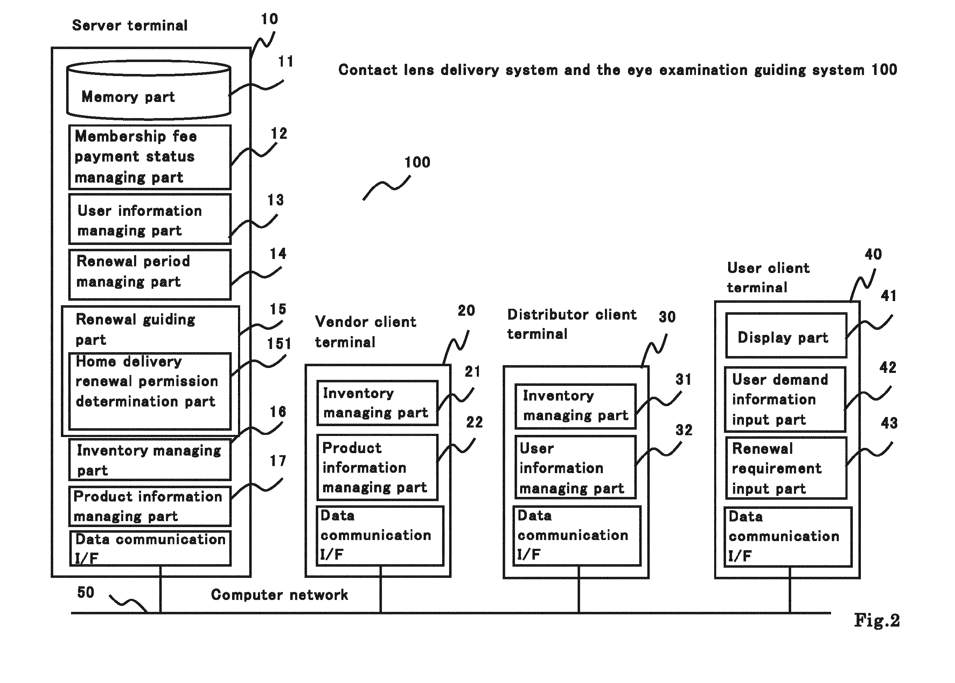

Fig.1 and Fig.2 are block diagrams describing the configuration of the embodiment 1 of the contact lens delivery system and the eye examination guiding system 100 simply.

As shown in Fig.1 and Fig.2, the contact lens delivery system and the eye examination guiding system 100 includes a server terminal 10, a vendor client terminal 20, a distributor client 30, a user terminal 40, and a computer network 50.

The server terminal 10 is a computer terminal used by the system control administrator of this system 100.

The server terminal 10 includes a memory 11, a membership fee payment status managing part 12, a user information managing part 13, a renewal period managing part 14, a renewal guiding part 15, an inventory managing part 16 and a product information managing part 17. The server terminal 10 serves functions of this system 100 using and controlling these elements.

The configuration shown in Fig.1 includes a home delivery renewal permission determination part 151 combined in the renewal guiding part 15, but another configuration shown in Fig.2 can be possible. In this configuration, a home delivery renewal permission determination part 151 is separated from the renewal guiding part 15.

The home delivery renewal permission determination part 151 can be provided as the combination part of the other element as shown in Fig.1 (Fig. 1 is the example that it is combined with the renewal guiding part 15), or can be provided as the independent element separated from other elements as shown in Fig.2. It may be designed according to the system requirement and processing content.

This embodiment is an example of the configuration that the home delivery renewal permission determination part 151 is provided as the combination part of the renewal guiding part 15.

The vendor client terminal 20 is a computer terminal used by each contact lens vendor who participates in this system 100.

In this embodiment1, there are several contact lens vendors who participate in this system 100, and every vendor has the vendor client terminal 20 (only one vendor client terminal is described as an example in Fig.1 and Fig.2). If there is only one contact lens vendor who participate in this system 100, this system 100 can be also applied.

The vendor client terminal 20 includes an inventory managing part 21 and a product information providing part 22.

The distributor client terminal 30 is a computer terminal used by each contact lens distributor store who participates in this system 100 (only one vendor client terminal is described as an example in Fig.1 and Fig.2).

The distributor client terminal 30 includes an inventory managing part 31 and a user information managing part 32.

The user client terminal 40 is a computer terminal used by each user who participates in this system 100. For example, the user client terminal may be a small terminal such as a personal computer or a mobile phone. The user client terminal 40 includes a display part 41, a user demand information input part 42, and a renewal requirement input part 43.

The computer network 50 may be a data communication network such as the internet or the telecommunication line.

In Fig.1, the system 100 includes a single terminal for each element. However, the number of each element is not limited. For example, the single vendor client terminal 20 or the plural vendor client terminal 20 may be employed. If the plural vendors are participating in this system 100, each vendor uses and manages each vendor client terminal 20, so there are plural vendor client terminals 20. For example, the single distributor client terminal 30 or the plural distributor client terminal 30 may be employed. If the plural distributors are participating in this system 100, each distributor uses and manages each distributor client terminal 30, so there are plural distributor client terminals 30. For example, the single user client terminal 40 or the plural user client terminal 40 may be employed. If the plural users are participating in this system 100, each user uses and manages each user client terminal 40, so there are plural user client terminals 40. These terminals are data-communicatively connected to each other by the computer network 50.

Hereinafter, these elements are described respectively, and then the process of the contact lens delivery service and the eye examination guiding by the contact lens delivery system and the eye examination guiding system 100 are described.

The elements of the server terminal 10 are described.

The memory 11 is data storage device of the server terminal 10. Various type of data storage device can be employed as the memory 11. For example, a silicon disk memory installed in the server terminal 10, a hard disk apparatus installed in the server terminal 10, or a storage device accessible on the network may be employed.

Next, the membership fee payment status managing part 12 is an element for storing the information of the periodical membership fee payment of the user to the system control administrator referencing with the user identification into the memory part 11 of the server terminal 10.

As described later, the contact lens delivery system and the eye examination guiding system 100 of the present invention assist the user to pay the membership fee periodically to the service control administrator and to take the contact lens renewal service. Therefore, the user payment of the membership fee to the system control administrator without delinquency of the payment can be verified. The contact lens delivery system and the eye examination guiding system 100 of the present invention verifies the status of the user payment of the membership fee by the membership fee payment status managing part 12 of the server terminal 10. The verification system is not limited. For example, the management staff of the system control administrator may input an inquiry of the status of the user payment of the membership fee via the input device, or may verify the status of the user payment of the membership fee by extracting the information of the user payment of the membership fee from the user banking transfer information to the system control administrator bank account by cooperating the banking system via the computer network 50.

Next, the user information managing part 13 is an element for processing the user information based on the diagnosis information obtained by the eye examination of the user at the contact lens distributor and the prescribed contact lens information with referencing to the user personal data. The processed information is stored in the memory part 11 of the server terminal 10 via the distributor client terminal 30. As described later, when the diagnosis information obtained by the eye examination of the user at the contact lens distributor and the prescribed contact lens information such as the type of the contact lens and the number of the handout contact lens is processed, the user information managing part 32 of the distributor client terminal 30 transmits these diagnosis information and the prescribed contact lens information to the user information managing part 13. The user information managing part 13 stores these information to the memory 11 and manages. As described later, the contact lens delivery system and the eye examination guiding system 100 of the present invention require the uniform management of the information regarding diagnosis information indicating the user eye health status and prescribed contact lens information in order to provide the cooperation among the contact lens vendors and the contact lens distributors. Therefore, the server terminal 10 collects the diagnosis information and the prescribed contact lens information for the uniform management by the system control administrator in order to avoid segregating these diagnosis information and the prescribed contact lens information by the contact lens distributor alone.

The renewal period managing part 14 is an element for accessing the memory 11 and informing the user client terminal 40 of the forthcoming of the renewal due date of the contact lens before predetermined days judging by the calculation result of the renewal due date based on the information such as payment information of the membership fee, diagnosis information and the prescribed contact lens information stored in the memory 11. The system can guide the renewal of the contact lens at an appropriate timing if the user is not conscious of the renewal period. This system can guide the actual appropriate renewal due date judged by the renewal period managing part 14 based on the diagnosis information managed uniformly by the server terminal 10, not based on the superficial information recommended by the contact lens vendor. Therefore, the system can guide a more appropriate renewal due date.

Next, the renewal guiding part 15 is an element for displaying and guiding the information such as information of the contact lens distributor and information of the currently use contact lens for renewal and information of other contact lens products for replacement based on the latest diagnosis information and the user demand information. As described later, the user can input the user demand information such as desired vendor, desired product, and desired the area of the contact lens distributor location via the user demand information input part 42 of the user client terminal 40. The renewal guiding part 15 displays the information on the display part 41 of the user client terminal 40 and guides the information such as information of the contact lens distributor, and information of the replaceable other contact lens products based on the user demand information and the latest diagnosis information.

The home delivery renewal permission determination part 151 is an element for judging permission of the utilization of the home delivery contact lens renewal according to the user demand information with diagnosis provided by the contact lens distributor based on the user eye status indicated in the latest diagnosis information and the term of the interval from the previous renewal date.

The home delivery renewal permission determination part 151 manages at least one rule regarding the judgment for permission of the utilization of the home delivery contact lens renewal according to the user demand information based on the user eye status indicated in the latest diagnosis information and the term of the interval from the previous renewal date. If there is the user demand for the home delivery contact lens renewal in the user demand information, the home delivery renewal permission determination part 151 judges whether or not to permit the home delivery contact lens renewal according to this rule.

The system 100 employs the home delivery renewal permission determination part 151 to judge the permission of the home delivery contact lens renewal, so the system 100 can judge appropriately. For example the system 100 can utilize the latest diagnosis information significantly to judge whether or not the user eye condition is easy to deteriorated, and manage the renewal due period to be shorter than the standard due period especially when the user demands the home delivery contact lens renewal.

When the user demands the home delivery contact lens renewal by inputting the user demand information, the renewal guiding part 15 judges whether or not to permit the home delivery contact lens renewal based on the judgment result of the home delivery renewal permission determination part 151. If the home delivery contact lens renewal is inputted, the renewal guiding part 15 displays the information of the replaceable contact lens product and the vendor on the user client terminal 40.

The home delivery renewal permission determination part 151 can display several inquiries on the user client terminal 40 and require the answer from the user before judging whether to permit the home delivery contact lens renewal. The home delivery renewal permission determination part 151 can consider the answer information from the user for judging whether to permit of the home delivery contact lens renewal.

The inventory managing part 16 is an element for managing the inventory information of the each contact lens product of each contact lens vendor. As described above, the renewal guiding part 15 decides the contact lens distributor from the view point of the ability for serving the diagnosis for the coming user and the sufficient inventory of desired contact lens product corresponding to the user demand before the renewal guiding part 15 sends the contact lens renewal guiding to the user client terminal 40. The inventory information is utilized for verifying the amount of the inventory of each contact lens product of each contact lens vendor in each distributor. Both the inventory information sent from the inventory managing part 21 of the vendor client terminal 20 used by the contact lens vendor and the inventory information sent from the inventory managing part 31 of the distributor client terminal 30 used by the contact lens distributor are received by the inventory managing part 16 of the server terminal 10 and all inventory information are managed uniformly.

The present system 100 processes the user demand by the cooperation among the contact lens vendors and the contact lens distributors beyond the affiliate relationship, and it is preferable that the inventory information of the contact lens vendor is collected and managed uniformly. Therefore, the inventory information of the contact lens vendor is transmitted from the inventory managing part 21 of the vendor client terminal 20 to the server terminal 10. The inventory information is managed by the system control administration who stands in a neutral position, so the contact lens vendors and the contact lens distributors can count on this present system 100.

The product information managing part 17 is an element for storing and managing the product information regarding the current contact lens products and newly developed contact lens products sent from the product information providing part 22 of the vendor client terminal 20 to the memory part 11. As described later, the vendor client terminal 20 includes the product information providing part 22 for transmitting the product information regarding the current contact lens products and newly developed contact lens products to the server terminal 10 and storing the information to the memory part 11. The system 100 manages the product information uniformly in the server terminal 10 in order to provide the product information regarding the current contact lens products and newly developed contact lens products by each contact lens vendor who participates to this system 100.

The renewal period managing part 14 accesses the product information managed by the product information managing part 17 and displays the forthcoming contact lens renewal due date and the recommended product information based on the user diagnosis information.

Next, the elements of the vendor client terminal 20 are described.

The inventory managing part 21 is an element for managing the inventory information of the each contact lens product of the contact lens vendor and sending the inventory information of the contact lens vendor to the server terminal 10 for storing in the memory part 11. It is preferable that the inventory information of each contact lens product of the contact lens vendor is managed uniformly in order to realize the user demand by cooperating the plural contact lens vendor and the plural contact lens distributor beyond the affiliate relationship without limiting the user. In order to achieve this cooperation, the vendor client terminal 20 send the inventory information of each contact lens vendor to the server terminal 10 via the inventory managing part 21.

The product information providing part 22 is an element for sending the product information regarding the current contact lens product and newly developed contact lens product to the server terminal 10 and storing and managing the product information to the memory part 11. This system works by cooperating among the plural contact lens vendor and the plural contact lens distributor beyond the affiliate relationship without enclosing the user. Therefore, it is preferable that the system provides the product information regarding the current contact lens product and newly developed contact lens product to the user corresponding to the user demand and the user diagnosis information. The server terminal 10 can manage the product information uniformly, and the server terminal 10 can inform the user of the forthcoming contact lens renewal due date by the renewal period managing part 14 and guide the user of the product information regarding the recommended contact lens based on the user diagnosis information via the user client terminal 40.

Next, the elements of the distributor client terminal 30 are described.

The distributor client terminal 30 includes the inventory managing part 31 and the user information managing part 32.

The inventory managing part 31 is an element for managing the inventory information of the each contact lens product of each contact lens vendor stored in each contact lens distributor and sending the inventory information of the contact lens distributor to the server terminal 10 for storing in the memory part 11. It is preferable that the inventory information of each contact lens distributor is managed uniformly in order to process the user demand by cooperating among the plural contact lens vendor and the plural contact lens distributor beyond the affiliate relationship without limiting the user. In order to achieve this cooperation, the distributor client terminal 30 send the inventory information of each contact lens distributor to the server terminal 10 via the inventory managing part 31.

The inventory managing part 31 can assist the renewal guiding part 15 of the server terminal 10 to select the contact lens distributor.

The user information managing part 32 is an element for sending the user information including the diagnosis information and the prescribed contact lens information obtained by the contact lens distributor referencing with the user identification to the server terminal 10 and storing the user information to the memory part 11. As described later, the contact lens information including the type and the number of the prescribed handed contact lens is obtained by the contact lens distributor. Therefore, the contact lens distributor inputs the diagnosis information and the contact lens information via the user information managing part 32 and sends the diagnosis information and the contact lens information as the user information referencing with the user identification information to the user information managing part 13 of the server terminal 10 via the computer network 50.

As described later, in the contact lens delivery system and the eye examination guiding system 100, the system control administrator manages uniformly the diagnosis information indicating the user eye status and the contact lens information indicating the currently prescribed contact lens information for the cooperation among the plural contact lens vendor and the plural contact lens distributor beyond the affiliate relationship without limiting the user. In order to achieve this cooperation, the contact lens distributor sends the information to the server terminal 10 but not enclose the diagnosis information and the contact lens information.

Next, the elements of the user client terminal 40 are described.

The user client terminal 40 includes the display part 41, the user demand information input part 42 and the renewal requirement input part 43.

The display part 41 is a monitor installed in the user client terminal 40 to display information. If the user client terminal 40 is a personal computer, the monitor apparatus can be used as the display part 41. If the client terminal 40 is a mobile phone, the liquid crystal display part installed in the mobile phone can be used as the display part 41.

The user demand information input part 42 is an element for receiving the input of the user demand information regarding the contact lens renewal including the replacement information for desired vendor and the desired product from the user client terminal 40. The user demand information is information such as desired vendor, desired product, and desired area of the contact lens distributor location. The user demand information input part 42 sends the user demand information to the server terminal 10. By the response to the sending of this user demand information, the user can obtain the replaceable contact lens vendor and the replaceable contact lens product and the contact lens distributor that corresponds to this new replacement based on the user demand information and the latest diagnosis information. The input device of the user demand information input part 42 is not limited to the particular device. Various input devices such as a keyboard, pointing device, and a touch panel can be employed.

The renewal requirement input part 43 is an element for inputting the renewal requirement information to require the contact lens renewal regardless of the normal periodical renewal due date. As a merit of the membership fee system, the user can require the contact lens renewal freely regardless of when the normal periodical renewal due date is. To input this user renewal requirement, the user client terminal 40 includes the renewal requirement input part 43 and sends the user renewal requirement information to the server terminal 10.

The input device of the renewal requirement input part 43 is not limited to the particular device. Various input devices such as a keyboard, pointing device, and a touch panel can be employed.

The renewal guiding part 15 of the server terminal 10 displays the replaceable contact lens vendor and the replaceable contact lens product and the contact lens distributor that correspond to this new renewal onto the display part 41 by answering to the transmitted user renewal requirement information from the user.

The contact lens delivery system and the eye examination guiding system 100 achieves functions by utilizing each element of the server terminal 10, the vendor client terminal 20, the distributor client terminal 30 and the user client terminal 40 as shown above.

Hereinafter, the basic process for contact lens delivery and eye examination guide performed by the contact lens delivery system and the eye examination guiding system 100 is described.

The process for the contact lens renewal is described by dividing into two parts. First, the process for the contact lens renewal by the system when the periodical contact lens renewal due date comes is described as part [A]. Next, the process for the contact lens renewal by the user voluntary requirement before the periodical contact lens renewal due date comes is described as part [B]. There are two cases of this user voluntary requirement. First is that the user visits the contact lens distributor store voluntarily. The second is that user inputs the user requirement via the user client terminal 40.

### [A] The process for the contact lens renewal by the system when the periodical contact lens renewal due date comes

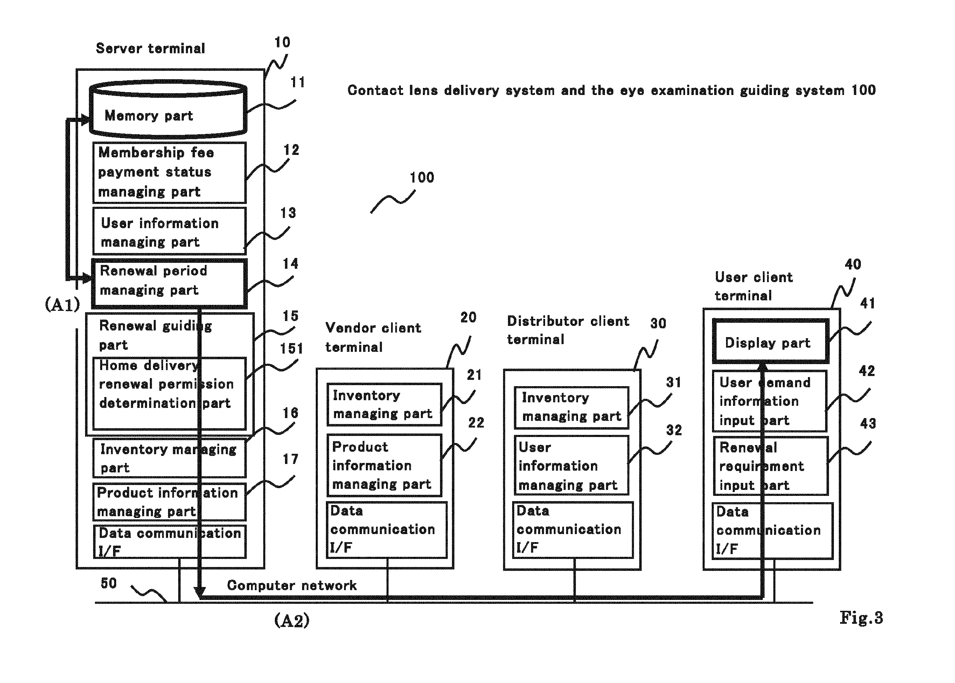

With referencing to Fig.3 to Fig.10, the basic process is shown for the contact lens renewal by the system when the periodical contact lens renewal due date comes. It is described by assuming that the user is the member of the system control administrator, the user pays monthly member fee without delinquency and the user is using the periodic renewal type contact lens.

### (A1) The schedule management of the periodical contact lens renewal by the renewal period managing part 14 (Fig.3)

The renewal period managing part 14 of the server terminal 10 accesses the data regarding the information for the user membership fee payment status, the diagnosis information and the contact lens information storing in the memory part 11. The renewal period managing part 14 calculates the periodical contact lens renewal due date of the currently used, contact lens and detects when the remaining period becomes equal to the predetermined period (the period is depend on the administration rule. For example, the predetermined period is one month). The renewal period managing part 14 verifies the user status of the membership fee payment and whether there is delinquency.

### (A2) The informing of the forthcoming of the periodical contact lens renewal due date to the user client terminal 40 (Fig.3)

The renewal period managing part 14 of the server terminal 10 generates the renewal guiding information for the periodical contact lens renewal and sends the renewal guiding information to the user client terminal 40 via the computer network 50 to display onto the display part 41 of the user client terminal 40.

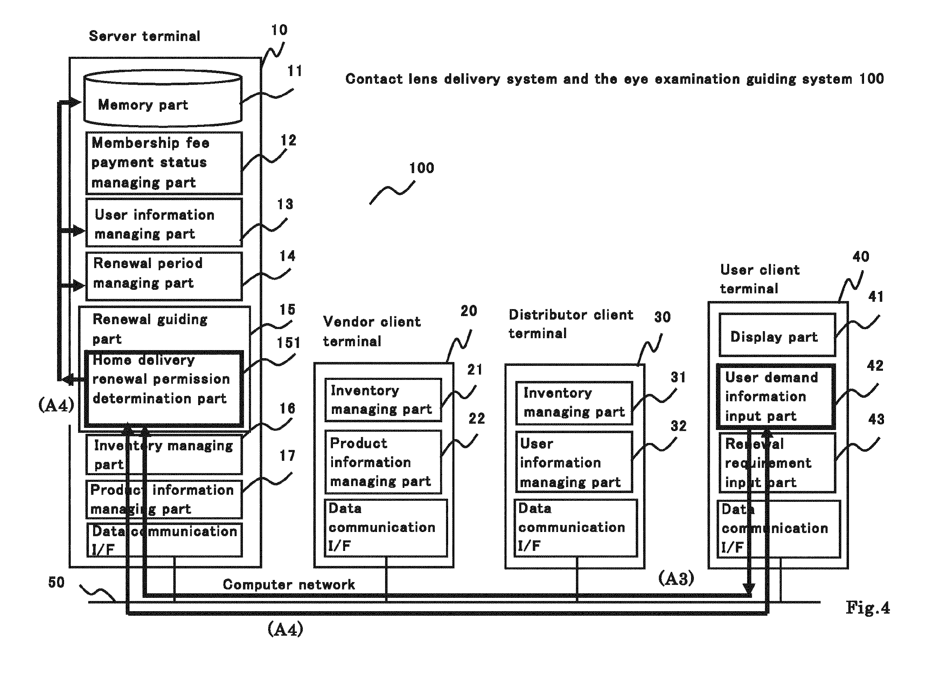

### (A3) The input of the user demand information via the user demand information input part 42 (Fig.4)

When the user watches the contact lens renewal guiding displayed on the display part 41 of the user client terminal 40, the user can input the user demand for the contact lens renewal via the user demand information input part 42. For example, the user inputs the user demand information including the replacement information of the desired vendor, the desired product. In addition, the user can select whether to utilize the home delivery contact lens renewal or visit the contact lens distributor store. If there is the user demand for utilizing the home delivery contact lens renewal, this user demand for the home delivery contact lens renewal is inputted to the home delivery renewal permission determination part 151. The user demand information input part 42 provides the input-form to assist the user to input user demand information such as the desired eye examination date, the desired store location, the desired vendor, and the desired product.

### (A4) The determination of the permission of the utilizing of the contact lens home delivery renewal by the home delivery renewal permission determination part 151 (Fig.4)

The home delivery renewal permission determination part 151 judges whether to permit of the utilization of the home delivery contact lens renewal without diagnosis provided by the contact lens distributor based on the user eye status indicated in the latest diagnosis information and the term of the interval from the previous renewal date. The home delivery renewal permission determination part 151 manages at least one judgment rule regarding the judgment whether or not to permit the utilization of the home delivery contact lens renewal by according to the judgment rule cooperating with the memory part 11, the user information managing part 13, and the renewal period managing part 14.

The home delivery renewal permission determination part 151 can display several inquiries on the display part 41 of the user client terminal 40 and require the answer from the user before determining whether to permit the home delivery contact lens renewal. The home delivery renewal permission determination part 151 can consider the answer information for judging whether or not to permit the home delivery contact lens renewal (A4).

The home delivery renewal permission determination part 151 judges the permission or refusal for the contact lens home delivery renewal and displays the judgment result information to the renewal guiding part 15.

The example of the judgment rules managed in the home delivery renewal permission determination part 151 and the basic process for using each judgment rule are described below.

There are five judgment rules, but they are examples and they can be employed alone single or in combination.

The first judgment rule managed by the home delivery renewal permission determination part 151 is the judgment from the interval term from the previous renewal date up to the recommended renewal due date based on the prescribed contact lens if the user uses the prescribed contact lens appropriately. If the interval term is too short from the previous renewal date to the user desired renewal date, there is a possibility that the user renews the disposal contact lens frequently because of the bad eye condition. On the contrary, if the interval term is too long from the previous renewal date to the user desired renewal date, there is a possibility that the user does not renew the contact lens according to the specified use period and keeps on inserting the current contact lens beyond the specified use period. For example, the user uses one-day disposable type contact lens, but the user frequently uses the same contact lens for two days consecutively. In those cases, the contact lens home delivery service should not be permitted without oculist eye examination. By this reason, the home delivery renewal permission determination part 151 can employ the rule considering the interval term from the previous renewal date as the first rule.

The renewal period managing part 14 manages the interval term from the previous renewal date up to the recommended renewal due date as the expecting interval term information. The home delivery renewal permission determination part 151 accesses the renewal period managing part 14 and obtains the expecting interval term information. If the interval term from the previous renewal date is longer or shorter than the predetermined term from the expecting interval term information, the home delivery renewal permission determination part 151 judges that this renewal does not satisfy the first judgment rule. However, there may be the circumstances such as that there is some no use term, or that the user has lost some contact lens. The home delivery renewal permission determination part 151 displays the inquiry on use of the contact lens on the display part 41 of the user client terminal 40 and waits the answer from the user. The home delivery renewal permission determination part 151 can consider the use by the user and the user circumstances for judging whether or not to permit of the home delivery contact lens renewal.

Next, the second judgment rule managed by the home delivery renewal permission determination part 151 is the judgment from the user's use experience of the desired contact lens product based on the prescription. There are various types of the contact lens according to the specification and the user's eye condition such as an oxygen permeable ratio, length of the periodic renewal term (one-day disposal, one-week disposal, two-week disposal or one-month disposal), and the quantity of the tear. Some contact lens are suitable for the particular user, and some contact lens are not suitable for the particular user.

If the user desired contact lens had been prescribed before and there was no problem for the user to use that desired contact lens, the home delivery renewal permission determination part 151 can estimate the user desired contact lens as the suitable type contact lens for the user. By this reason, the home delivery renewal permission determination part 151 can employs the rule considering the user's use experience based on the prescription as the second judgment rule.

User information managing part 13 manages the contact lens information prescribed in the past, so the home delivery renewal permission determination part 151 accesses and references the prescribed contact lens information managed by the user information managing part 13 and judges the permission of the home delivery renewal on the condition that the user desired product is the contact lens product prescribed to the user before. If the user desired product is not the contact lens product prescribed to the user before, the home delivery renewal permission determination part 151 judges the current demand does not satisfy the second judgment rule. If so, the renewal guiding part 15b displays the guide information on the display part 41 of the user client terminal 40 for the user to take to the oculist eye examination at the contact lens distributor store.

Next, the third judgment rule managed by the home delivery renewal permission determination part 151 is the judgment whether the desired contact lens product is from the vendor the user has used for other contact lens products before.

The same as the second rule, there are various types of the contact lens according to the specification and the user's eye condition, if the type of the desired contact lens is the same type as the currently use contact lens, but the condition may be different among vendors. The condition depends on the vendor. Therefore, the fact that the desired contact lens product is from the vendor the user has used before is treated as the third judgment rule. The same as the second judgment rule, the home delivery renewal permission determination part 151 accesses and references the prescribed contact lens information managed by the user information managing part 13 and judges whether to permit of the home delivery renewal on the condition that the vendor of the user desired product is the vendor the user has used for other products before. If the vendor of the user desired product is not the vendor the user has used for other contact lens products before, the home delivery renewal permission determination part 151 judges the current demand does not satisfy the third judgment rule. If so, the renewal guiding part 15b displays the guide information on the display part 41 of the user client terminal 40 for the user to take to the oculist eye examination at the contact lens distributor store.

Next, the fourth judgment rule managed by the home delivery renewal permission determination part 151 is the judgment from the validity of the prescription issued by the oculist. The user eye condition varies from day to day, and the latest diagnosis information may become unsuitable to estimate the user eye condition as the day passes from the latest eye examination by the oculist. Therefore, the validity of the prescription of the oculist is limited to judge whether the elapsed time from the latest eye examination by the oculist has passed the predetermined period. This rule is treated as the fourth judgment rule.

For example, the home delivery renewal permission determination part 151 manages the validity of the prescription by the oculist. If there is the demand for the home delivery contact lens renewal included in the user demand information, the home delivery renewal permission determination part 151 judges the permission of the home delivery renewal on whether the elapsed time from the latest eye examination by the oculist is over the predetermined validity of the prescription or not. If the elapsed time from the latest eye examination by the oculist is over the predetermined validity of the prescription, the home delivery renewal permission determination part 151 judges the current demand does not satisfy the fourth judgment rule. If so, the renewal guiding part 15b displays the guide information on the display part 41 of the user client terminal 40 for the user to take to the oculist eye examination at the contact lens distributor store.

Next, the fifth judgment rule managed by the home delivery renewal permission determination part 151 is the judgment from the user eye condition, especially the density of the corneal endothelium cell count of user eye. The status of the user eye condition varies during use of the contact lens. The number of the corneal endothelium cell count of user eye may decrease if the user eye condition is bad or the misuse of the contact lens occurred. In addition, aging leads to the decrease of the number of the corneal endothelium cell count of the user's eye.

Many contact lenses have high ratio of the oxygen permeability, but the user's eye falls into lack of oxygen depending on the eye condition. In other cases, the user eye falls into the tear deficiency because the tear does not go into between the corneal and the contact lens. In these cases, the number of the corneal endothelium cell count of user eye may decrease with the long term use of the contact lens. If the density of the corneal endothelium cell count of user eye is low, the eye examination is recommended. Therefore, the user eye condition including the density of the corneal endothelium cell count of user eye is treated as the fifth judgment rule.

For example, the home delivery renewal permission determination part 151 manages the threshold value of the density of the corneal endothelium cell count of user eye for permitting the home delivery contact lens renewal.

For example, the threshold value of the density of the corneal endothelium cell count of user eye is set as 2500/mm2. If the density of the corneal endothelium cell count of user eye equals to or is larger than 2500/mm2, the home delivery contact lens renewal is permitted. If the density of the corneal endothelium cell count of user eye is smaller than 2500/mm2, the home delivery contact lens renewal may be permitted on the condition that the user take an eye examination by the oculist and the oculist prescribes the contact lens to the user.

If there is the demand for the home delivery contact lens renewal included in the user demand information, the home delivery renewal permission determination part 151 accesses and references the prescribed contact lens information managed by the user information managing part 13 and judges the permission of the home delivery renewal on the condition that the density of the corneal endothelium cell count of user eye equals to or is larger than the threshold. In this example, the density of the corneal endothelium cell count of user eye equals to or is larger than 2500/mm2, and the home delivery renewal permission determination part 151 permits the home delivery contact lens renewal as the fifth judgment rule is satisfied. If the density of the corneal endothelium cell count of user eye is smaller than 2500/mm2, the home delivery renewal permission determination part 151 judges the home delivery contact lens renewal may be permitted on the condition that the user take an eye examination by the oculist and the oculist prescribes the contact lens to the user, and the renewal guiding part 15b displays the guide information on the display part 41 of the user client terminal 40 for the user to take to the oculist eye examination at the contact lens distributor store.

As shown above, the judgment rules managed by the home delivery renewal permission determination part 151 are explained, these judgment rules can be selected one by one or combined variously. In addition to those rules, other judgment rules can be employed to judge the permission of the home delivery contact lens renewal.

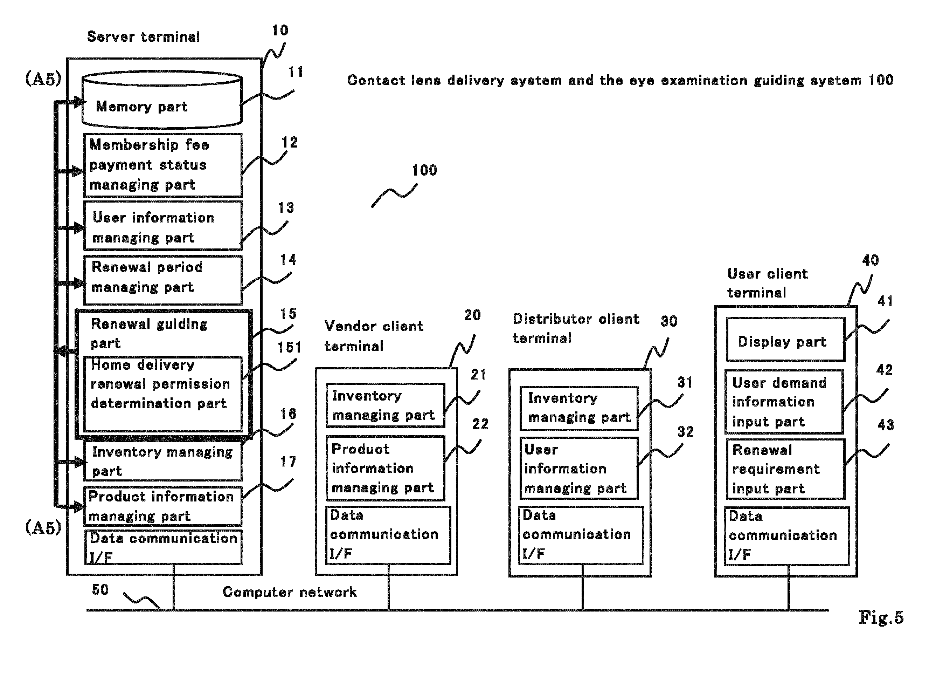

### (A5) The determination of the selectable several options of the replaceable contact lens and conditions by the renewal guiding part 15 (Fig.5)

The renewal guiding part 15 receives the permission judgment of the home delivery contact lens renewal, and the renewal guiding part 15 determines the replaceable contact lens products of the contact vendors corresponding to the user demand information inputted by the user demand information input part 42 of the user client terminal 40. The renewal guiding part 15 accesses various information managed by the memory 11, the membership fee payment status managing part 12, the user information managing part 13, the renewal period managing part 14, the inventory managing part 16, and the product information managing part 17 for determining the replaceable contact lens products of the contact vendors. For example, the renewal guiding part 15 checks the status of the membership fee payment and determines the recommended contact lens products of the recommended vendors based on the information such as the user demand information inputted by the user client terminal 40, the user information managed by the user information managing part 13, the contact lens product information managed by the product information managing part 17 and the inventory information managed by the inventory managing part 16.

If the renewal guiding part 15 receives the judgment information not permitting the home delivery contact lens renewal from the home delivery renewal permission determination part 151, the renewal guiding part 15 determines the replaceable contact lens products of the contact vendors corresponding to the user demand information inputted by the user demand information input part 42 of the user client terminal 40. In this case, the information shows that the home delivery contact lens renewal is not permitted, and the information that replaceable contact lens product of the contact vendors with taking eye examination instead of the user desired contact lens product of the contact vendors. For example, the renewal guiding part 15 determines the proposed reservation day of the eye examination, the proposed reservation clinic of the oculist, the recommended vendor and the recommended contact lens product.

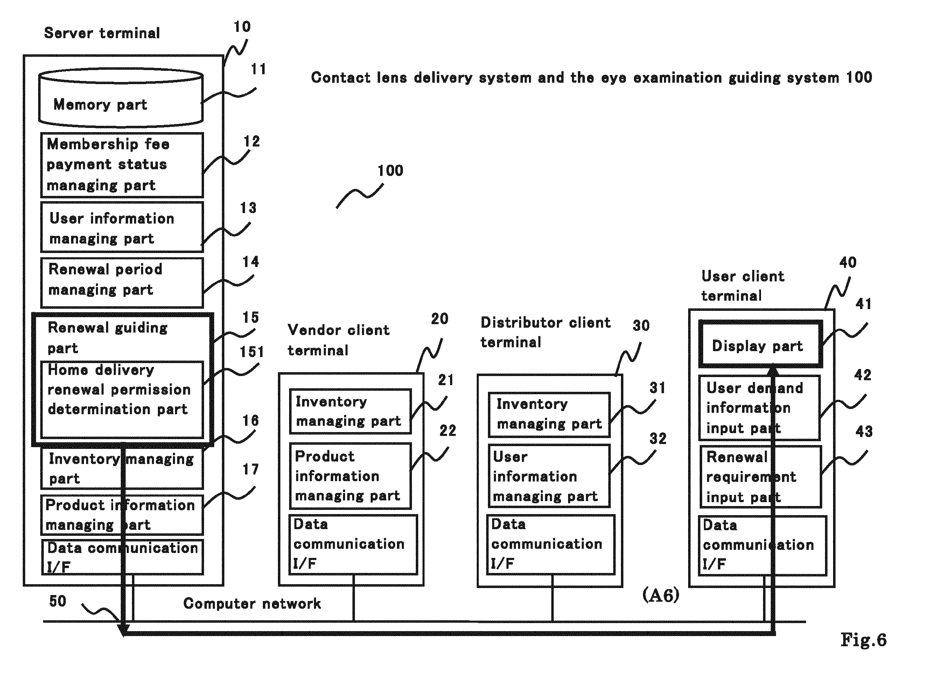

### (A6) The display of the selectable several options of the replaceable contact lens and conditions by the renewal guiding part 15 (Fig.6)

The renewal guiding part 15 sends and displays the information for the selectable several options of the replaceable contact lens and conditions onto the display part 41 of the user client terminal 40 via the computer network 50.

If the home delivery contact lens renewal is permitted, the information regarding the home delivery service such as the scheduling of the delivery date is displayed in addition to the information for the selectable several options of the replaceable contact lens and conditions.

If the home delivery contact lens renewal is not permitted, the information regarding the eye examination such as the proposed reservation day of the eye examination, the proposed reservation clinic of the oculist, the recommended vendor and the recommended contact lens product are displayed onto the display part 41 of the user client terminal 40.

If the user is not able to visit oculist clinic on schedule for some reason, the user should make a reservation for the eye examination again, and the user may repeat the processing from (A1) to (A5) shown above.

Regarding the processing after (A6) processing, the flow is divided into two flows. One is the case the home delivery contact lens renewal is permitted, the other is the case the home delivery contact lens renewal is not permitted. Each processing for both cases is explained separately as follows.

There are two types of providers of the contact lens in the home delivery contact lens renewal. One is the contact lens distributor, the other is the contact lens vendors. In the following explanation, the contact lens distributor provides the home delivery contact lens renewal via the distributor client terminal 30, but if the contact lens vendor provides the home delivery contact lens renewal, the flow will be the same as the contact lens distributor case except for using the vendor client terminal 20 instead.

The case when the home delivery contact lens renewal is permitted is described as follows.

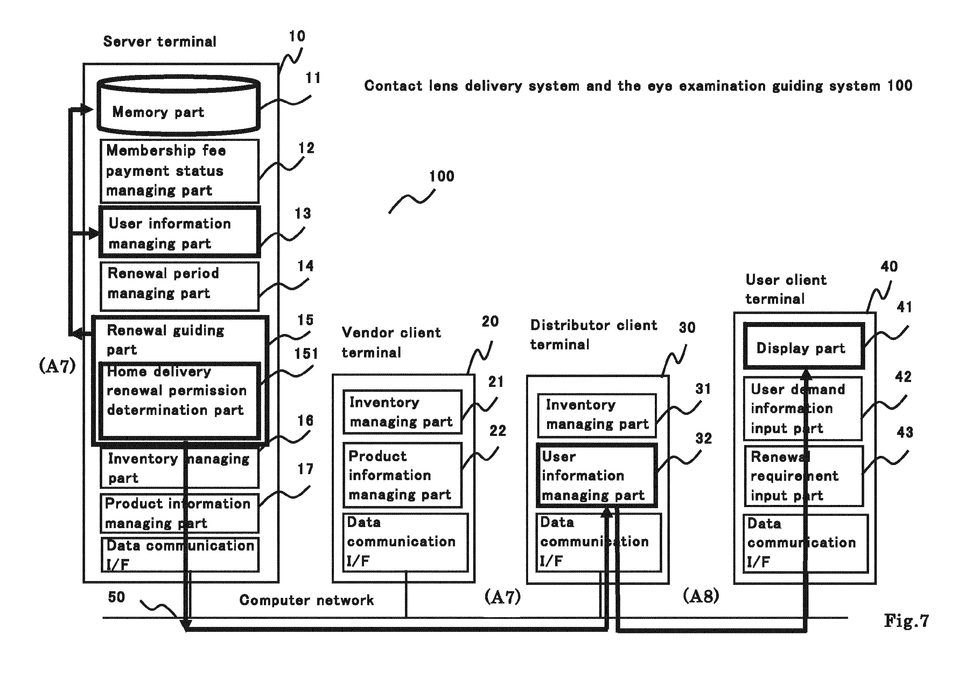

### (A7) The informing of the information regarding the home delivery contact lens renewal (Fig.7)

The renewal guiding part 15 sends the information regarding the home delivery contact lens renewal decided by the communication result with the user to the user information managing part 32 of the distributor client terminal 30 (A7).

The new contact lens distributor different from the previous contact lens distributor selected in the previous renewal may be selectable. Therefore, the renewal guiding part 15 should collect the latest diagnosis information cooperating with the user information managing part 13 and send the latest information to the user information managing part 32 of the distributor client terminal 30.

The user information managing part 32 of the distributor client terminal 30 receives the information regarding the home delivery contact lens renewal and verifies the inventory cooperating with the inventory managing part 31, and then displays the information regarding the home delivery contact lens renewal for the staff to dispatch.

### (A8) The display regarding the home delivery service on the user client terminal 40 (Fig.7)

The distributor client terminal 30 may display the information regarding the home delivery contact lens renewal onto the display part 41 of the user client terminal to inform the user of the home delivery service.

The report of the accomplishment of the dispatch by the home delivery service is sent from the distribution client terminal 30 to the server terminal 10.

Next, the case where the home delivery contact lens renewal is not permitted is described as follows.

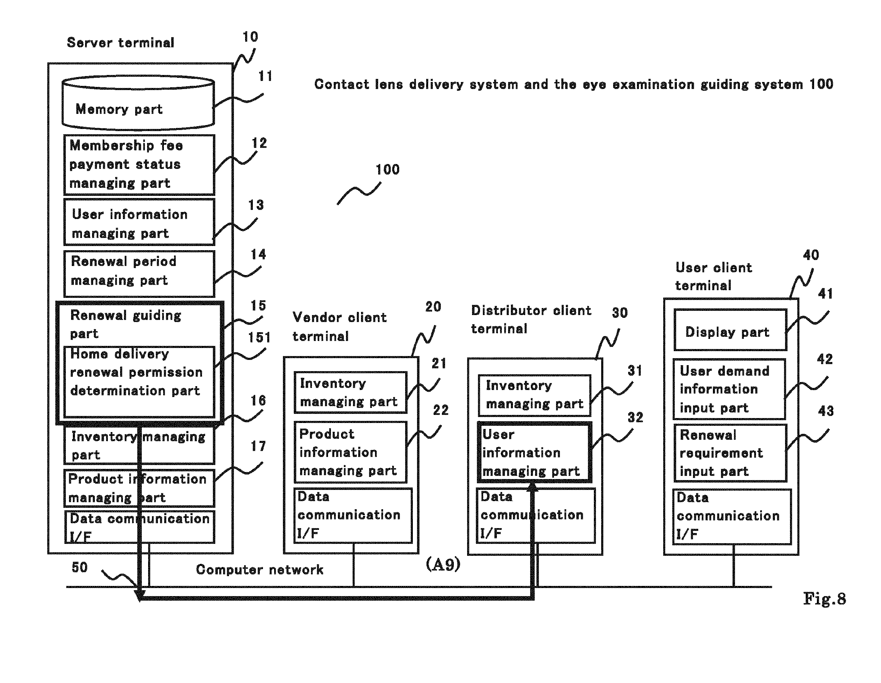

### (A9) The informing of the reservation information for the exe examination to the contact lens distributor (Fig.8)

The renewal guiding part 15 informs the reservation information for the exe examination and the user demand information for contact lens renewal to the user information managing part 32 of the distributor client terminal 30 managed by the contact lens distributor who is selected by the user.

The new contact lens distributor different from the previous contact lens distributor selected in the previous eye examination may be selectable. Therefore, the renewal guiding part 15 should collect the latest diagnosis information cooperating with the user information managing part 13 and send the latest information to the user information managing part 32 of the distributor client terminal 30.

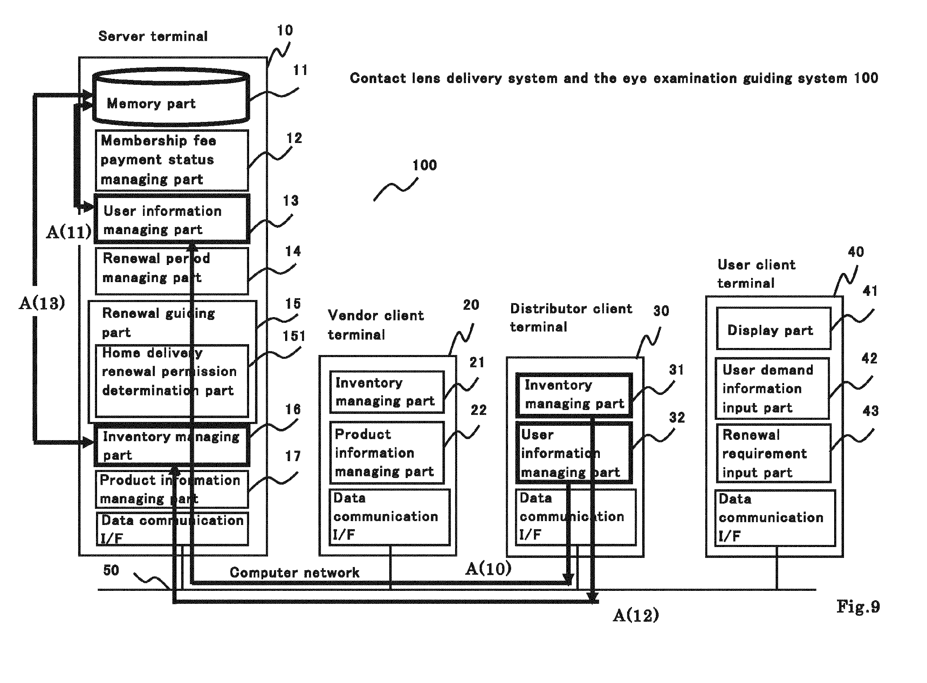

### (A10) The eye examination process and the contact lens renewal process by the contact lens distributor (Fig.9)

The user visits the contact lens distributor store informed by the system control administrator via the server terminal 10 and takes the reserved eye examination and obtains the new contact lens according to the prescription based on the diagnosis result. The contact lens distributor sends the information of the diagnosis and the information of the prescribed contact lens from the user information managing part 32 of the distributor client terminal 30 to the server terminal 10 via the computer network 50.

### (A11) The uniform management of the user information by the server terminal 10 (Fig.9)

The user information managing part 13 of the server terminal 10 receives the user information from the distributor client terminal 30 and storing in the memory part 11. The distributor client terminal 30 may transmit the user information each time or in bulk once a day.

### (A12) The report on the inventory at the contact lens distributor (Fig.9)

The inventory at the contact lens distributor may vary from day to day, and the inventory information is reported from the inventory managing part 31 of the distributor client terminal 30 to the server terminal 10 via the computer network 50.

### (A13) The uniform management of the information of the inventory at the contact lens distributor by the server terminal 10 (Fig.9)

The inventory managing part 16 manages the information of the inventory at the contact lens distributor received from the distributor client terminal 30 to the memory part 11. The distributor client terminal 30 may transmit the inventory information each time or in bulk once a day.

### (A14) The report of the information of the inventory at the contact lens vendor to the

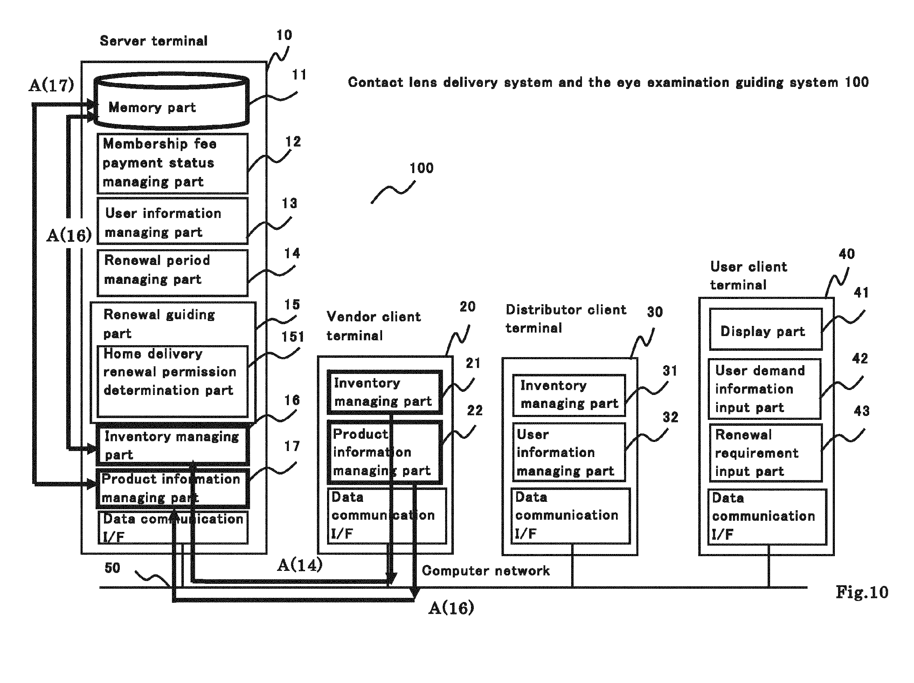

### server terminal 10 (Fig. 10)

The inventory at the contact lens vendor may vary from day by day, the inventory information is reported from the inventory managing part 21 of the vendor client terminal 20 to the server terminal 10 via the computer network 50.

### (A15) The uniform management of the information of the inventory at the contact lens vendor by the server terminal 10 (Fig.9)

The inventory managing part 16 manages the information of the inventory at the contact lens vendor received from the vendor client terminal 20 to the memory part 11. The vendor client terminal 20 may transmit the inventory information each time or in bulk once a day.

### (A16) The report of the product information of the contact lens vendor to the server terminal 10 (Fig.10)

In order to maintain high quality service to the user, the product information providing part 22 of the vendor client terminal 20 sends the current contact lens product and newly available contact lens product to the server terminal 10 via the computer network 50.

### (A17) The uniform management of the contact lens product information by the server terminal 10 (Fig.10)

The product information managing part 17 manages the contact lens product information received from the vendor client terminal 20 to the memory part 11. The contact lens product information managed uniformly by the product information managing part 17 is utilized in the determination processing of the selectable contact lens options by the renewal guiding part 15. The renewal guiding part 15 may display the selectable contact lens options based on the diagnosis information onto the display part 41 of the user client terminal 40.

Those are the basic processing flows of the home delivery contact lens renewal and the eye examination guide when the notice of the periodic renewal is sent by the server terminal 10 to the user client terminal based on the periodic management.

Next, the process for the contact lens renewal by the user voluntary requirement before the periodical contact lens renewal due date comes is described as follows.

### [B] The process for the contact lens renewal by the user voluntary requirement

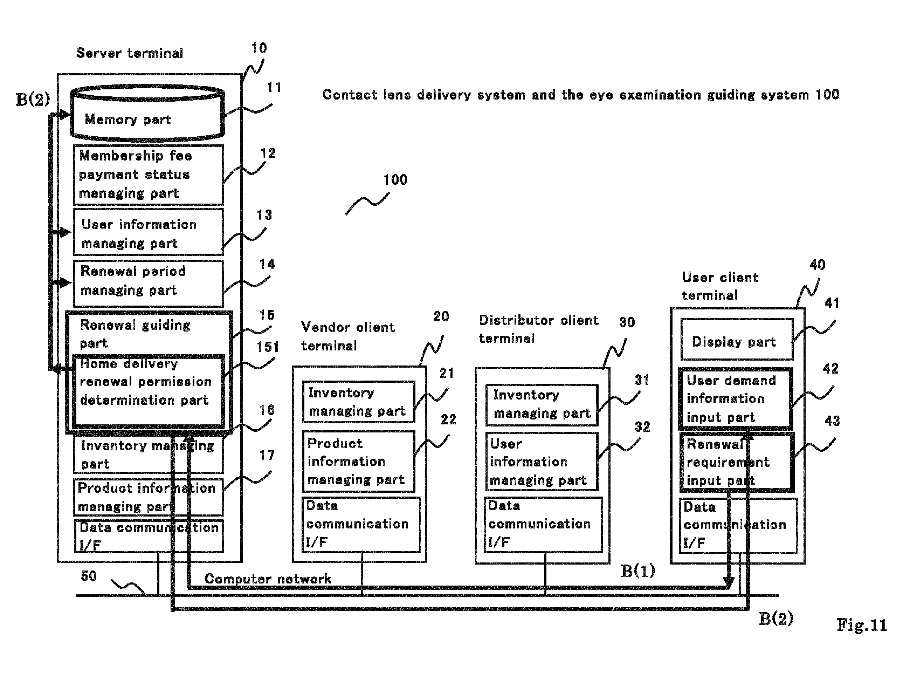

With reference to Fig.11 to Fig.12, the basic process for the contact lens renewal by the user voluntary requirement before the periodical contact lens renewal due date comes is described. It is described by assuming that the user is the member of the system control administrator, the user pays monthly membership fee without delinquency and the user is using the periodic renewal type contact lens.

### (B1) The input of the user voluntary requirement via the renewal requirement input part 43 (Fig.11)

The user can input the user voluntary requirement for current use contact lens renewal for new contact lens via the renewal requirement input part 43 of the user client terminal 40 to the server terminal 10 regardless of the periodic contact lens renewal due date. The same process as A3, the user can input the user demand for the contact lens renewal via the user demand information input part 42. For example, the user inputs the user demand information including the replacement information for desired vendor and the desired product. In addition, the user can select whether to utilize the home delivery contact lens renewal or visit the contact lens distributor store.

The user demand information input part 42 provides the input-form to assist the user to input user demand information such as the desired eye examination date, the desired area, the desired vendor, and the desired product.

If there is the user demand for utilizing the home delivery contact lens renewal, this user demand for the home delivery contact lens renewal is inputted to the a home delivery renewal permission determination part 151.

If the user voluntarily visits the contact lens distributor store and demands the contact lens renewal verbally, the staff can input the user demand and the necessary information instead via the distributor client terminal 30.

### (B2) The determination of the permission of the utilizing of the contact lens home delivery renewal by the home delivery renewal permission determination part 151 (Fig.11)

The same process as A4, the home delivery renewal permission determination part 151 judges whether to permit the utilization of the home delivery contact lens renewal without diagnosis provided by the contact lens distributor based on the user eye status indicated in the latest diagnosis information and the term of the interval from the previous renewal date.

The home delivery renewal permission determination part 151 manages at least one judgment rule regarding the judgment whether or not to permit the utilization of the home delivery contact lens renewal according to the judgment rule by cooperating with the memory part 11, the user information managing part 13, and the renewal period managing part 14.

The home delivery renewal permission determination part 151 can display the several inquiries on the display part 41 of the user client terminal 40 and require the answer from the user before determining whether to permit the home delivery contact lens renewal. The home delivery renewal permission determination part 151 can consider the answer information for judging whether to permit the home delivery contact lens renewal (B2).

The home delivery renewal permission determination part 151 judges the permission or refusal for the contact lens home delivery renewal and displays the judgment result information to the renewal guiding part 15.

In the above example, there are five judgment rules, but they are examples and they can be employed single or in combination.

### (B2) The determination of the selectable several options of the replaceable contact lens and conditions by the renewal guiding part 15 (Fig.11)

The renewal guiding part 15 receives the permission judgment of the home delivery contact lens renewal, the renewal guiding part 15 determines the replaceable contact lens products of the contact vendors corresponding to the user voluntary requirement via the renewal requirement input part 43

The renewal guiding part 15 accesses various information managed by the memory 11, the membership fee payment status managing part 12, the user information managing part 13, the renewal period managing part 14, the inventory managing part 16, and the product information managing part 17 for determining the replaceable contact lens products. For example, the renewal guiding part 15 checks the status of the membership fee payment and determines the recommended contact lens products of the recommended vendors based on the information such as the user voluntary requirement, the user information managed by the user information managing part 13, the contact lens product information managed by the product information managing part 17 and the inventory information managed by the inventory managing part 16.

If the renewal guiding part 15 receives the judgment information not for permitting the home delivery contact lens renewal from the home delivery renewal permission determination part 151, the renewal guiding part 15 determines the replaceable contact lens products of the replaceable contact vendors corresponding to the user demand information inputted by the user demand information input part 42 of the user client terminal 40. In this case, the information shows that the home delivery contact lens renewal is not permitted, and the replaceable contact lens product of the contact vendors instead of the user desired contact lens product of the contact vendors. For example, the renewal guiding part 15 determines the proposed reservation day of the eye examination, the proposed reservation clinic of the oculist, the recommended vendor and the recommended contact lens product.

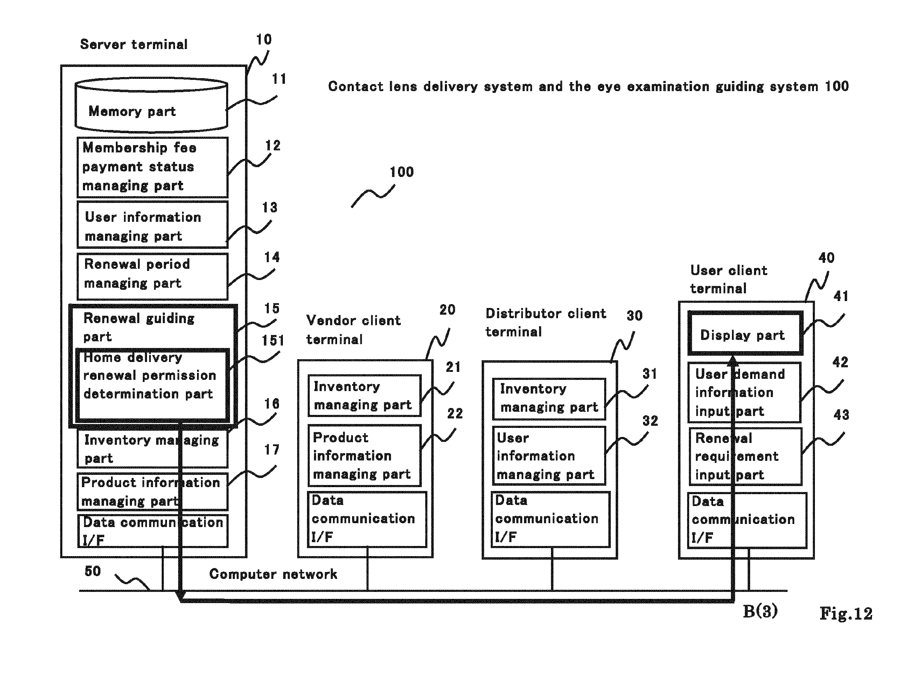

### (B3) The display of the selectable several options of the replaceable contact lens and conditions by the renewal guiding part 15 (Fig.12)

The renewal guiding part 15 sends and displays the information for the selectable several options of the replaceable contact lens and conditions onto the display part 41 of the user client terminal 40 via the computer network 50.

If the home delivery contact lens renewal is permitted, the information regarding the home delivery service such as the scheduling of the delivery date is displayed in addition to the information for the selectable several options of the replaceable contact lens and conditions.

If the home delivery contact lens renewal is not permitted, the information regarding the eye examination such as the proposed reservation day of the eye examination, the proposed reservation clinic of the oculist, the recommended vendor and the recommended contact lens product are displayed onto the display part 41 of the user client terminal 40.

If the user is not able to visit oculist clinic on schedule for somewhat reason, the user should make a reservation for the eye examination again, and the user may repeat the processing from (B1) to (B2) shown above.

As shown above, if the user voluntarily visits the contact lens distributor store and demands the contact lens renewal verbally, the staff may input the user demand and the necessary information instead via the distributor client terminal 30. In this case, the renewal guiding part 15 can display the information onto the display part 41 of the user client terminal 40 directly or onto the monitor of the distributor client terminal 30 by return.

The processes after the reservation for visiting the contact lens distributor store are the same as (A6) to (A14), so the duplicative description is omitted here.

The processes when the user voluntarily visits the distributor store and demands the contact lens renewal verbally, after the input process for the user demand information via the distributor client terminal 30 and the display process for the replaceable contact lens information by the renewal guiding part 15 are the same as (A6) to (A14), so the duplicative description is omitted here.

### Embodiment 2

The embodiment 2 of the configuration of the contact lens delivery system and the eye examination guiding system 100a is described below.

The embodiment 2 of this system is added the element for cooperating with the system of the public health insurance for the whole nation to the basic configuration shown in embodiment 1.

All Japanese people join the system of the public health insurance for the whole nation, and the eye examination for wearing the contact lens by an oculist is applicable to the public health insurance. The contact lens delivery system and the eye examination guiding system 100a are used in conformity with laws and regulations, so all users are estimated as the insuree of the public health insurance. If the contact lens distributor notices that the visiting user lost the qualification as the insuree of the public health insurance, this irregular service affects the system operations of the present invention of the contact lens delivery system and the eye examination guiding system 100a. This embodiment 2 of the contact lens delivery system and the eye examination guiding system 100a is reinforced for the cooperation with the data base system of the public health insurance for the whole nation system.

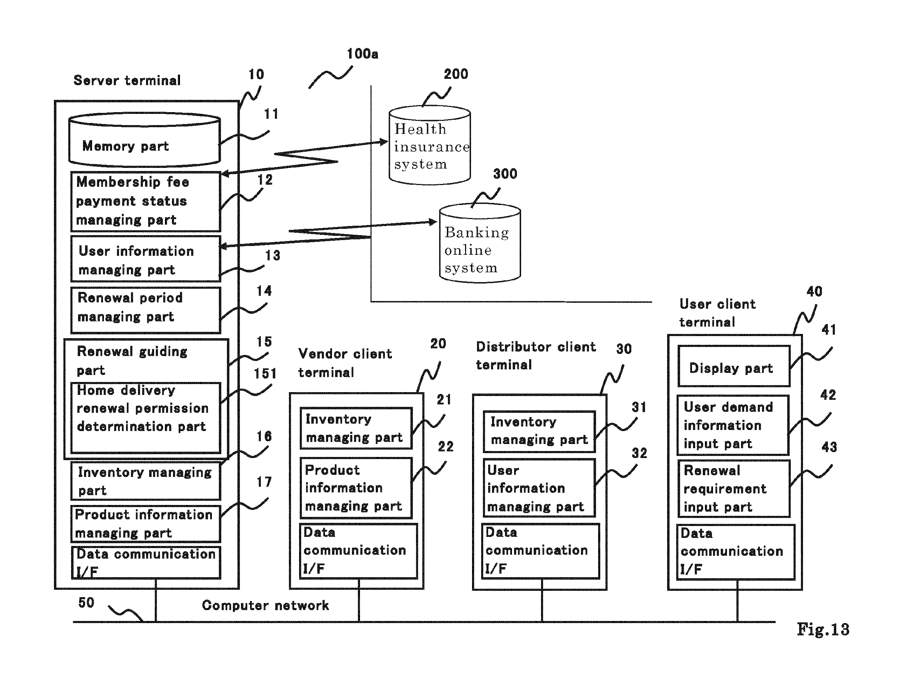

Fig.13 is the block chart describing the configuration of the embodiment 2 of the contact lens delivery system and the eye examination guiding system 100a simply. As shown in Fig.13, the embodiment 2 of the contact lens delivery system and the eye examination guiding system 100a further includes an insuree qualification information managing part 18 in addition to the membership fee payment status managing part 12, and cooperates with the national health insurance system 200 and the banking on-line system 300. Other configurations are the same as those shown in embodiment 1, so the detail descriptions are omitted.

The insuree qualification information managing part 18 is an element for obtaining the insuree qualification information of the user from the national health insurance system 200 and storing the obtained information to the memory part 11 with referencing to the user personal information. For example, the server terminal 10 and the national health insurance system 200 are connected via the computer network 50.

Hereinafter, the basic process for contact lens delivery and eye examination guide performed by the embodiment 2 of the contact lens delivery system and the eye examination guiding system 100a is described.

### [C] The process for the contact lens renewal cooperating with the insuree qualification information

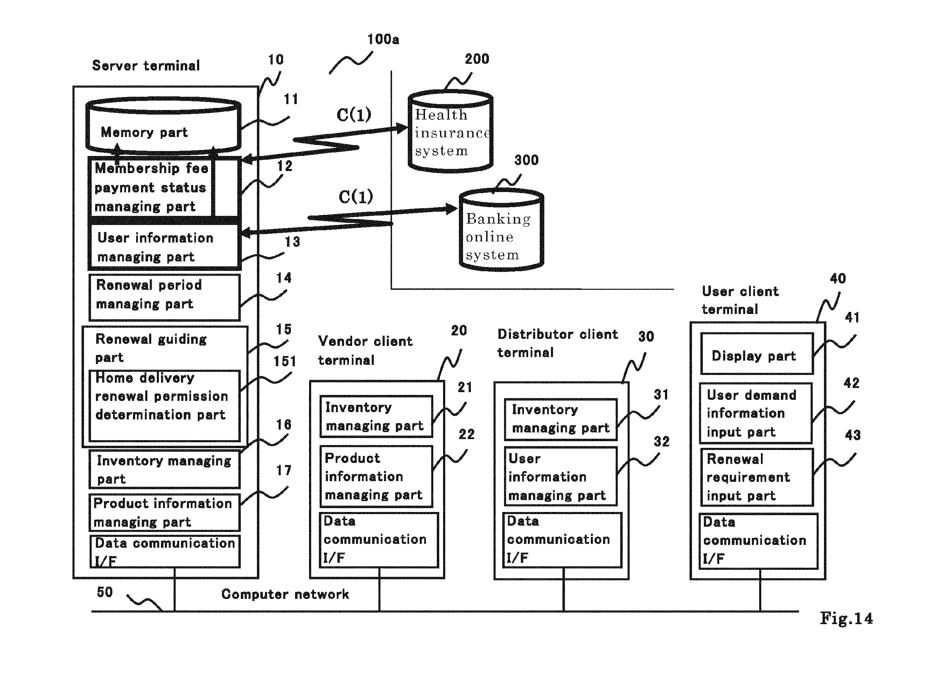

With reference to Fig.14 to Fig.15, the basic process for the contact lens renewal cooperating with the insuree qualification information.

### (C1) Uniform management of the insuree qualification information by the insuree qualification information managing part 18 (Fig.14)

As shown in Fig.14, the insuree qualification information managing part 18 obtains the insuree qualification information of each user from the national health insurance system 200 and stores in the memory part 11 with reference to the user personal data.

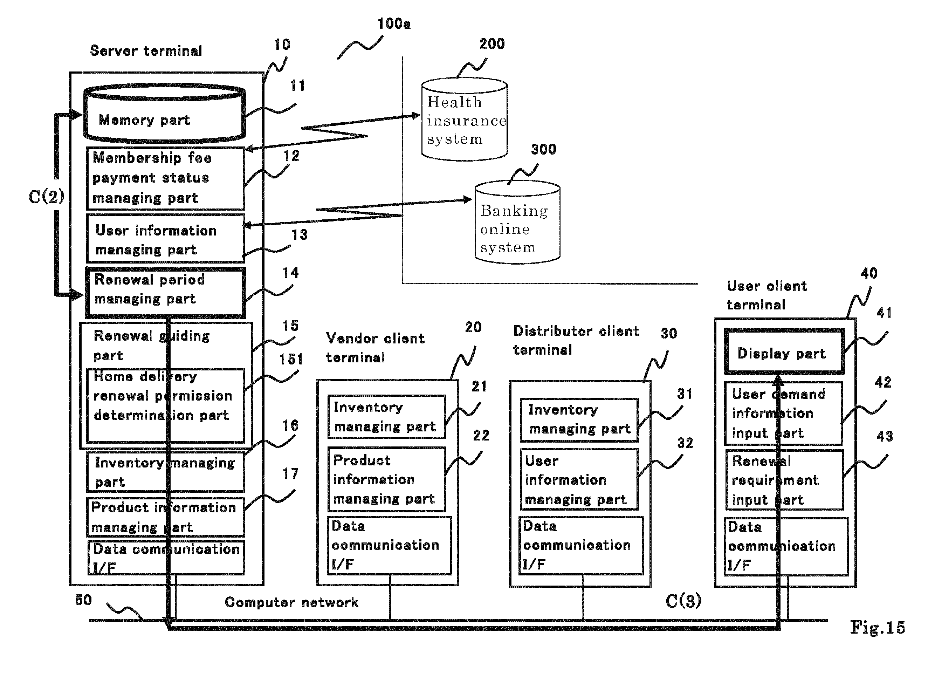

### (C2) The schedule management of the contact lens renewal by the renewal period managing part 14 (Fig.15)

The renewal period managing part 14 of the server 10 access the insuree qualification data in addition to the membership fee payment information, diagnosis information and the contact lens information stored in the memory part 11. The renewal period managing part 14 can detect the forthcoming contact lens renewal due date by calculating the remaining term of the currently use contact lens by the user at hand and whether the remaining term become equal to or shorter than the predetermined period (For example, one month). The renewal period managing part 14 verifies no delinquency of the membership fee payment and the no suspension of the insuree qualification. The renewal period managing part 14 informs and guides the contact lens renewal only if no delinquency of the membership fee payment and no suspension of the insuree qualification are verified.

### (C3) The informing the forthcoming contact lens renewal to the user client terminal 40 by the renewal period managing part 14 (Fig. 15)

The renewal period managing part 14 of the server terminal 10 generates the renewal guiding information for the periodical contact lens renewal and sends the renewal guiding information to the user client terminal 40 via the computer network 50 to display onto the display part 41 of the user client terminal 40.

In this embodiment 2, the verification of no delinquency of the membership fee payment and the no suspension of the insuree qualification is the condition for the renewal period managing part 14 to send the renewal guiding information, and there will be no confusion for the eye examination in the contact lens distributor store whether the national health insurance is applicable to the user.

If the insuree qualification of the national health insurance is not verified, the renewal period managing part 14 may display the information that the user has not paid the national health insurance premium and display the guide information for payment of the national health insurance premium instead of displaying the guide information for the contact lens renewal. There is a possibility that the user just forget the payment of the national health insurance premium,and the display of the guide information for the contact lens renewal is a preferable process for user friendliness and the system operation.

The processes following to the process of the information for the user of the forthcoming contact lens renewal are the same as (A3) to (A14), so the duplicative description is omitted here.

While some preferable embodiments of the contact lens delivery system and the eye examination guiding system according to the present invention are described above, it should be understood that various changes are possible, without deviating from the technical scope according to the present invention. Therefore, the technical scope according to the present invention is limited only by the claims attached.

### Industrial applicability

The contact lens delivery system and the eye examination guiding system according to the present invention can be used as the system which can guide and assist the user who uses the disposable type contact lens to be informed of the appropriate periodical contact lens renewal and to have a suitable new contact lens based on the appropriate eye examination.

### Brief description of the drawings

Fig.1 is a block diagram describing the configuration of the embodiment 1 of the contact lens delivery system and the eye examination guiding system 100 (part 1).
Fig.2 is a block diagram describing the configuration of the embodiment 1 of the contact lens delivery system and the eye examination guiding system 100 (part 2).
Fig.3 is a block diagram describing a basic process for the contact lens delivery system and the eye examination guiding system (part 1).
Fig.4 is a block diagram describing a basic process for the contact lens delivery system and the eye examination guiding system (part 2).
Fig.5 is a block diagram describing a basic process for the contact lens delivery system and the eye examination guiding system (part 3).
Fig.6 is a block diagram describing a basic process for the contact lens delivery system and the eye examination guiding system (part 4).
Fig.7 is a block diagram describing a basic process for the contact lens delivery system and the eye examination guiding system (part 5).
Fig.8 is a block diagram describing a basic process for the contact lens delivery system and the eye examination guiding system (part 6).
Fig.9 is a block diagram describing a basic process for the contact lens delivery system and the eye examination guiding system (part 7).
Fig.10 is a block diagram describing a basic process for the contact lens delivery system and the eye examination guiding system (part 8).
Fig.11 is a block diagram describing a basic process for the contact lens delivery system and the eye examination guiding system (part 9).
Fig.12 is a block diagram describing a basic process for the contact lens delivery system and the eye examination guiding system (part 10).
Fig.13 is a block diagram describing the configuration of the embodiment 2 of the contact lens delivery system and the eye examination guiding system 100a.
Fig.14 is a block diagram describing a basic process for the contact lens delivery system and the eye examination guiding system 100a (part 1).
Fig.15 is a block diagram describing a basic process for the contact lens delivery system and the eye examination guiding system 100a (part 2).
Fig.16 is a block diagram describing the configuration of the conventional contact lens renewal system.

### The reference number in the Figs.

- 100, 100a, 100b: denotes a contact lens delivery system and the eye examination guiding system
- 10: denotes a server terminal
- 11: denotes a memory
- 12: denotes a membership fee payment status managing part
- 13: denotes a user information managing part
- 14: denotes a renewal period managing part
- 15: denotes a renewal guiding part
- 151: denotes a home delivery renewal permission determination part
- 16: denotes an inventory managing part
- 17: denotes a product information managing part
- 18: denotes an insuree qualification information managing part
- 20: denotes a vendor client terminal
- 21: denotes an inventory managing part
- 22: denotes a product information providing part
- 30: denotes a distributor client terminal
- 31: denotes an inventory managing part
- 32: denotes a user information managing part
- 40: denotes a user client terminal
- 41: denotes a display part
- 42: denotes a user demand information input part
- 43: denotes a renewal requirement input part
- 50: denotes a computer network
- 200: denotes a health insurance system
- 300: denotes a banking online system

## Claims

1. A contact lens delivery system and the eye examination guiding system for managing the delivery of a contact lens which has a predetermined validation period for renewal and guiding the user for taking the eye examination, comprising;
a server terminal utilized by a system control administrator;
a vendor client terminal used by a contact lens vendor;
a distributor client terminal used by each contact lens distributor;
a user client terminal used by each user;
a computer network providing data connection among those terminals;
a user information managing part for processing the user information based on the diagnosis information obtained by the user eye examination and the prescribed contact lens information with referencing to the user personal data, and storing the processed data in the memory part of the server terminal;
a renewal period managing part for informing the user client terminal of the forthcoming contact lens renewal due date based on the diagnosis information and the prescribed contact lens information stored in the memory part;
a user demand information input part for receiving the user demand regarding the contact lens renewal from the user client terminal;
a home delivery renewal permission determination part for judging the permission of the utilization of the home delivery contact lens renewal without eye examination based on the user eye status indicated in the latest diagnosis information and the interval from the previous renewal date;
a renewal guiding part for displaying the information of the replaceable contact lens products by the home delivery renewal on the user client terminal on the condition that the home delivery renewal permission determination part judges the permission of the home delivery contact lens renewal when the user demand for the home delivery is included in the user demand information.

2. A contact lens delivery system and the eye examination guiding system for managing the delivery of the contact lens which has predetermined validation period for renewal and guiding the user for taking the eye examination, comprising;
a server terminal utilized by a system control administrator;
a vendor client terminal used by each contact lens vendor;
an each distributor client terminal used by each contact lens distributor;
an each user client terminal used by each user;
a computer network providing data connection among those terminals;
a user information managing part for processing the user information based on the diagnosis information obtained by the user eye examination and the prescribed contact lens information with referencing to the user personal data, and storing the processed data in the memory part of the server terminal;
a renewal period managing part for informing the user client terminal of the forthcoming contact lens renewal due date based on the diagnosis information and the prescribed contact lens information stored in the memory part;
a user demand information input part for receiving the user demand regarding the contact lens renewal including the replacement information for desired vendor and the desired product from the user client terminal;
a home delivery renewal permission determination part for judging the permission of the utilization of the home delivery contact lens renewal without eye examination based on the user eye status indicated in the latest diagnosis information and the interval from the previous renewal date;
a renewal guiding part for displaying the information of the replaceable contact lens product of each vendor based on the latest diagnosis and the user demand information regarding the contact lens renewal on the user client terminal on the condition that the home delivery renewal permission determination part judges the permission of the home delivery contact lens renewal when the user demand for the home delivery contact lens renewal in the user demand information.

3. A contact lens delivery system and the eye examination guiding system according to claim 1 or 2, wherein the home delivery renewal permission determination part displays the inquiry how the user uses the current contact lens on the user client terminal when the length of the interval term from the previous renewal date is longer or shorter than the expected interval term, and receives the answer from the user to judge the permission of the home delivery contact lens renewal considering the answer information of how the user use the current contact lens.

4. A contact lens delivery system and the eye examination guiding system according to any one of claims 1 to 3, wherein the home delivery renewal permission determination part accesses and references the prescribed contact lens information managed by the user information managing part and judges the permission of the home delivery renewal on the condition that the user desired product is the contact lens product which has prescribed to the user before.

5. A contact lens delivery system and the eye examination guiding system according to any one of claims 1 to 4, wherein the home delivery renewal permission determination part accesses and references the prescribed contact lens information managed by the user information managing part and judges the permission of the home delivery renewal on the condition that the user takes newly eye examination at the contact lens distributor store if the user desired product is not the contact lens product prescribed to the user before.

6. A contact lens delivery system and the eye examination guiding system according to any one of claims 1 to 5, wherein the home delivery renewal permission determination part manages the validity of the prescription by the oculist and judges the permission of the home delivery renewal on the condition that user to take to the oculist eye examination at the contact lens distributor store, if there is the demand for the home delivery contact lens renewal included in the user demand information and if the validity period is over from the latest eye examination by the oculist.

7. A contact lens delivery system and the eye examination guiding system according to any one of claims 1 to 6, wherein the home delivery renewal permission determination part manages the threshold value of the density of the corneal endothelium cell count of user eye for permitting the home delivery contact lens renewal and judges the permission of the home delivery renewal on the condition that the density of the corneal endothelium cell count of user eye equals to or is larger than the threshold, if there is the demand for the home delivery contact lens renewal included in the user demand information.

8. A contact lens delivery system and the eye examination guiding system according to any one of claims 1 to 7, further comprising;
a membership fee payment status managing part for storing the information of the periodical membership fee payment of the user to the system control administrator referencing with the user identification into the memory part of the server terminal, the renewal period managing part informs the forthcoming contact lens renewal due date on the condition that the renewal period managing part can verify the user status of the membership fee payment without delinquency.

9. A contact lens delivery system and the eye examination guiding system according to any one of claims 1 to 8, further comprising;
an inventory managing part for managing the inventory information of the each contact lens product of each contact lens vendor, the renewal guiding part utilizing the inventory information for determining the selection of the contact lens distributor.

10. A contact lens delivery system and the eye examination guiding system according to any one of claims 1 to 9, further comprising;
a renewal requirement input part for inputting the renewal requirement information to require the contact lens renewal regardless of the normal periodical renewal due date, if the renewal guiding part receives the renewal requirement information, the renewal guiding part displays and guides the information of the contact lens distributor and contact lens products for renewal for corresponding to the user voluntarily renewal requirement.

11. A contact lens delivery system and the eye examination guiding system according to any one of claims 1 to 10, wherein the demand for the area of the contact lens distributor location is included in the user demand information, the user demand information input part utilizes the area information for listing up the selectable contact lens distributors.

12. A contact lens delivery system and the eye examination guiding system according to any one of claims 1 to 10, wherein the server terminal informs the latest diagnosis information and the user demand information to the selected contact lens distributor in advance of the user visiting schedule, the contact lens distributor being selected among the selectable contact lens distributors displayed on the user client terminal by the renewal guiding part

13. A contact lens delivery system and the eye examination guiding system according to any one of claims 1 to 12, wherein
the vendor client terminal includes a product information providing part for providing the current contact lens products and the newly developed contact lens products and storing in the memory part of the server terminal;
the server terminal includes product information managing part for managing the contact lens product information stored in the memory part; and
the renewal period managing part informs the forthcoming contact lens renewal due date and the renewal guiding part displays the product information of the recommended contact lens product based on the diagnosis information.

14. A contact lens delivery system and the eye examination guiding system according to any one of claims 1 to 13, further comprising;
an insuree qualification information managing part for storing the insuree qualification information of the national health insurance to the memory part with referencing to the user personal information,
the renewal period managing par informs the forthcoming contact lens renewal due date to the user client terminal on condition that verifying the no suspension of the insuree qualification.

15. A contact lens delivery system and the eye examination guiding system according to claim 14, if the renewal period managing part cannot verify the no suspension of the insuree qualification of the national health insurance, the renewal period managing part may display the guide information for payment of the national health insurance premium instead of displaying the guide information for the contact lens renewal.
